(19) Europäisches Patentamt / European Patent Office / Office européen des brevets

(11) EP 2 437 737 B1

(12) # FASCICULE DE BREVET EUROPEEN

(45) Date de publication et mention de la délivrance du brevet:
**29.08.2018 Bulletin 2018/35**

(21) Numéro de dépôt: **10724790.0**

(22) Date de dépôt: **07.06.2010**

(51) Int Cl.:
***A61K 31/015*** (2006.01) ***A61K 31/045*** (2006.01)
***A61K 31/08*** (2006.01) ***A61K 31/11*** (2006.01)
***A61K 31/22*** (2006.01) ***A61K 31/352*** (2006.01)
***A61K 31/37*** (2006.01) ***A61K 45/06*** (2006.01)
***A61P 31/04*** (2006.01)

(86) Numéro de dépôt international:
**PCT/EP2010/057905**

(87) Numéro de publication internationale:
**WO 2010/139805 (09.12.2010 Gazette 2010/49)**

(54) **COMPOSITION COMPRENANT AU MOINS DU TRANS-CINNAMALDEHYDE ET SON UTILISATION DANS LE TRAITEMENT DES INFECTIONS BACTERIENNES PLUS PARTICULIEREMENT DANS LE TRAITEMENT DES MALADIES NOSOCOMIALES**

ZUSAMMENSETZUNGEN MIT MINDESTENS EINEM TRANS-CINNAMALDEHYD UND IHRE VERWENDUNG ZUR BEHANDLUNG VON BAKTERIELLEN INFEKTIONEN, IM BESONDEREN ZUR BEHANDLUNG VON NOSOKOMIALEN INFEKTIONEN

COMPOSITION INCLUDING AT LEAST ONE TRANS-CINNAMALDEHYDE AND THE USE THEREOF IN THE TREATMENT OF BACTERIAL INFECTIONS, SPECIFICALLY IN THE TREATMENT OF NOSOCOMIAL INFECTIONS

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **05.06.2009 FR 0953734**

(43) Date de publication de la demande:
**11.04.2012 Bulletin 2012/15**

(73) Titulaire: **Septeos**
**75017 Paris (FR)**

(72) Inventeur: **TESSE, Nicolas**
**92420 Vaucresson (FR)**

(74) Mandataire: **Regimbeau**
**20, rue de Chazelles**
**75847 Paris Cedex 17 (FR)**

(56) Documents cités:
**WO-A-01/07094 WO-A-2007/109804**
**US-A- 4 477 361 US-A1- 2008 131 533**

- **SHAHVERDI A R ET AL: "Trans-cinnamaldehyde from Cinnamomum zeylanicum bark essential oil reduces the clindamycin resistance of Clostridium difficile in vitro" JOURNAL OF FOOD SCIENCE, WILEY-BLACKWELL PUBLISHING, INC, US, vol. 72, no. 1, 1 janvier 2007 (2007-01-01), pages S055-S058, XP002472467 ISSN: 0022-1147**
- **SENHAJI O ET AL: "Inactivation of Escherichia coli O157:H7 by essential oil from Cinnamomum zeylanicum" BRAZILIAN JOURNAL OF INFECTIOUS DISEASES, CONTEXTO, BARRA, BR LNKD- DOI:10.1590/S1413-86702007000200013, vol. 11, no. 2, 1 avril 2007 (2007-04-01), pages 234-236, XP002514011 ISSN: 1413-8670**
- **EZZAOUIA S ET AL: "Investigation of essential oils to fight multiresistant bacteria in hygienicand therapeutic applications" INTERNATIONAL JOURNAL OF ESSENTIAL OIL THERAPEUTICS, CHURCHILL LIVINGSTONE, GB, vol. 1, no. 2, 1 janvier 2007 (2007-01-01) , pages 51-55, XP009096945**
- **DE BILLERBECK V -G: "Essential oils and antibiotic-resistant bacteria" PHYTOTHERAPIE 200712 FR, vol. 5, no. 5, décembre 2007 (2007-12), pages 249-253, XP002555219**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- DATABASE WPI Week 200277 Thomson Scientific, London, GB; AN 2002-710791 XP002593857 & JP 2002 256300 A (LION CORP) 11 septembre 2002 (2002-09-11)
- SACCHETTI G ET AL: "Essential oil of wild Ocotea quixos (Lam.) Kosterm. (Lauraceae) leaves from Amazonian Ecuador" FLAVOUR AND FRAGRANCE JOURNAL JULY/AUGUST 2006 JOHN WILEY AND SONS LTD GB, vol. 21, no. 4, juillet 2006 (2006-07), pages 674-676, XP002593858 DOI: DOI:10.1002/FFJ.1648
- LEE HAN-CHUNG ET AL: "Antifungal property of the essential oils and their constituents from Cinnamomum osmophloeum leaf against tree pathogenic fungi" JOURNAL OF THE SCIENCE OF FOOD AND AGRICULTURE, vol. 85, no. 12, septembre 2005 (2005-09), pages 2047-2053, XP002593859 ISSN: 0022-5142
- MAYAUD L ET AL: "Comparison of bacteriostatic and bactericidal activity of 13 essential oils against strains with varying sensitivity to antibiotics." LETTERS IN APPLIED MICROBIOLOGY SEP 2008, vol. 47, no. 3, septembre 2008 (2008-09), pages 167-173, XP002555217 ISSN: 1472-765X
- CHANG S T ET AL: "Antibacterial activity of leaf essential oils and their constituents from Cinnamomum osmophloeum." JOURNAL OF ETHNOPHARMACOLOGY SEP 2001, vol. 77, no. 1, septembre 2001 (2001-09), pages 123-127, XP002555218 ISSN: 0378-8741
- ALI N A M ET AL: "Chemical composition and antimicrobial activities of the essential oils of Cinnamomum aureofulvum Gamb" JOURNAL OF ESSENTIAL OIL RESEARCH, XX, XX, vol. 14, no. 2, 1 mars 2002 (2002-03-01), pages 135-138, XP009125688 ISSN: 1041-2905
- RATTANACHAIKUNSOPON PONGSAK ET AL: "Potential of cinnamon (Cinnamomum verum) oil to control Streptococcus iniae infection in tilapia (Oreochromis niloticus)" FISHERIES SCIENCE (TOKYO), vol. 76, no. 2, mars 2010 (2010-03), pages 287-293, XP002593860
- CHENG S S ET AL: "Chemical polymorphism and antifungal activity of essential oils from leaves of different provenances of indigenous cinnamon (Cinnamomum osmophloeum)", BIORESOURCE TECHNOLOGY, ELSEVIER BV, GB, vol. 97, no. 2, 1 January 2006 (2006-01-01), pages 306-312, XP027965442, ISSN: 0960-8524 [retrieved on 2006-01-01]


Remarques:

Le dossier contient des informations techniques présentées postérieurement au dépôt de la demande et ne figurant pas dans le présent fascicule.

## Description

La présente invention concerne le domaine des antibactériens. Elle concerne plus précisément une composition, notamment antibactérienne ou pharmaceutique, notamment utilisée dans le cadre du traitement des infections bactériennes, tout particulièrement induites par des bactéries résistantes, en particulier en milieu hospitalier.

L'arrivée de la pénicilline, puis de la streptomycine vers les années 40 a ouvert l'ère des antibactériens. L'introduction de la pénicilline suivie de nombreux autres agents antimicrobiens, comme les aminosides, bêtalactamines, macrolides et quinolones, a représenté l'un des plus grands succès de la médecine moderne dans le traitement des infections bactériennes.

Cependant, le besoin de nouvelles molécules permettant de lutter contre les infections d'origine bactérienne est grandissant. En effet, notamment en santé humaine les infections nosocomiales constituent des infections graves qui affectent les patients hospitalisés. Elles s'avèrent particulièrement redoutables pour les patients dont le système immunitaire est fragilisé, par exemple suite à des traitements comme la corticothérapie ou la chimiothérapie, des interventions comme des transplantations ou des maladies affectant le système immunitaire telles le Syndrome d'Immunodéficience Acquise (SIDA). La croissance de la fréquence et de la sévérité des infections nosocomiales coïncide avec l'émergence de bactéries résistantes aux antibactériens existants.

A titre d'exemples de bactéries à Gram positif présentant un phénomène de pharmacorésistance, on peut notamment citer les bactéries de genre *Staphylococcus* en particulier *Staphylococcus aureus,* et les bactéries de genre *Enterococcus,* en particulier *Enterococcus faecalis.*

A titre d'exemples de bactéries à Gram négatif présentant un phénomène de pharmacorésistance, on peut notamment citer les bactéries de genre *Escherichia,* en particulier *Escherichia coli,* les bactéries de genre *Pseudomonas,* en particulier *Pseudomonas aeruginosa*, et les bactéries de genre *Acinetobacter,* en particulier *Acinetobacter baumanii.*

Un exemple préoccupant est celui de *Staphylococcus aureus.* En effet, plus de 95% des souches bactériennes *Staphylococcus aureus* sont résistantes à la pénicilline et plus de 60% sont également devenues résistantes à son dérivé, la méthicilline. Des souches présentant une sensibilité réduite à la vancomycine ont encore été caractérisées.

Selon l'Organisation Mondiale de la Santé, le taux de souches *Staphylococcus aureus* méthicilline-résistantes devenues résistantes à la mipirocine, un antibactérien inhibant la synthèse protéique, a progressé de 2,7% à 65% en l'espace de 3 ans. Ceci montre que l'action d'analogues d'antibactériens conventionnels peut être rapidement contrée par des mécanismes de résistances multiples élaborés par les bactéries.

Le phénomène de pharmaco-résistance est d'autant plus inquiétant qu'il n'est plus confiné au secteur hospitalier mais tend à se disséminer au sein de la communauté. On observe en effet une prévalence élevée des infections causées par *Staphylococcus aureus* chez les personnes âgées de 65 ans et plus. La proportion d'infections bactériennes, pneumonies, endocardites, infections bactériennes ostéoarticulaires ou urinaires développées par les personnes âgées et liées à *Staphylococcus aureus,* est tout particulièrement préoccupante.

Les bacilles à Gram négatif, notamment les entérobactéries et *Pseudomonas aeruginosa*, sont naturellement résistants, le plus souvent à bas niveau, aux antibactériens hydrophobes et/ou de masse moléculaire élevée (pénicilline G, pénicilline M, macrolides, rifampicine, acide fusidique, novobiocine, vancomycine) car ces antibactériens ne peuvent traverser la membrane externe de la paroi.

L'apparition et la propagation de souches bactériennes résistantes à pratiquement tous les agents antimicrobiens disponibles actuellement deviennent un problème de santé majeur.

Il existe donc un besoin grandissant de caractériser et d'obtenir de nouvelles classes de molécules anti-bactériennes, plus particulièrement actives contre les bactéries à Gram positif et/ou les bactéries à Gram négatif, si possible actives à la fois contre les bactéries à Gram positif et les bactéries à Gram négatif.

Un des buts de l'invention est donc l'obtention d'agents antibactériens actifs contre un large spectre de bactéries, en particulier contre des souches résistantes. Selon une variante ces agents bactériens sont actifs sur des souches présentant des résistances non naturelles. Selon une autre variante, ces agents bactériens sont actifs sur des souches présentant des résistances naturelles.

La définition de résistance naturelle peut notamment être trouvée dans les pages 15, 16 et 17 du Communiqué 2006 (Edition de janvier 2006) du Comité de l'antibiogramme de la Société Française de Microbiologie.

En pathologie infectieuse, une bactérie est dite « résistante » lorsque la concentration d'antibactérien qu'elle est capable de supporter est notablement plus élevée que celle qu'il est possible d'obtenir *in vivo* à la suite d'un traitement.

Un des buts de l'invention est également la détermination d'agents antibactériens présentant une forte activité, en particulier à faible dose, vis-à-vis de leurs cibles.

Parmi les autres buts de l'invention, on peut citer l'obtention d'agents antibactériens peu onéreux, faciles à préparer, présentant un spectre d'activité large, voire très large, présentant une activité antifongique, actifs même en présence d'agents interférents, ne déclenchant pas ou peu de résistances, en particulier de résistances croisées, et/ou qui soient bien tolérés dans et/ou sur l'organisme à traiter.

Les inventeurs ont découvert, de manière inattendue, que ces buts pouvaient être atteints, en tout ou partie,

en utilisant une composition comprenant de l'aldéhyde trans-cinnamique, encore appelé trans-cinnamaldéhyde.

L'aldéhyde trans-cinnamique ou trans-cinnamaldéhyde (CAS 14371-10-9) répond à la formule chimique suivante :

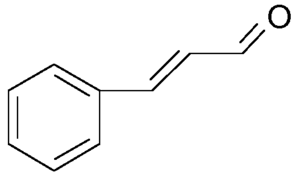

Il peut comprendre des quantités non négligeables, pouvant aller jusqu'à un rapport racémique, de son isomère l'aldéhyde cis-cinnamique.

Cependant, l'aldéhyde trans-cinnamique peut comprendre moins de 20 % en poids, notamment moins de 10 % en poids, en particulier moins de 5 % en poids, voire moins de 2 % en poids, tout particulièrement moins de 1 % en poids de cis-cinnamaldéhyde, voire être dépourvu de cis-cinnamaldéhyde.

Ainsi, selon un premier aspect, l'invention a pour objet une composition pour son utilisation dans le traitement ou la prévention des infections bactériennes comprenant, de l'aldéhyde trans-cinnamique.

Cette composition antibactérienne est telle que définie à la revendication 1 et comprend outre le trans-cinnamaldéhyde, au moins un composé choisi parmi le trans-2-méthoxycinnamaldéhyde, l'acétate de cinnamyle, la coumarine, le linalol, le béta-caryophyllène, l'eugénol, le cinéole, le benzoate de benzyle et le safrole, voire au moins un composé choisi parmi le trans-2-méthoxycinnamaldéhyde, l'acétate de cinnamyle, la coumarine, le linalol et le béta-caryophyllène.

Tout particulièrement, la composition antibactérienne comprend, voire consiste en, au moins quatre, et encore plus particulièrement au moins cinq des composés énoncé ci-dessus.

La composition antibactérienne comprend, au moins des composants différents choisis chacun respectivement dans un des trois groupes suivants :

- trans-cinnamaldéhyde,
- au moins un composé choisi parmi l'acétate de cinnamyl, le trans-2-méthoxycinnamaldéhyde, l'eugénol et la coumarine, notamment l'acétate de cinnamyle, ici au moins l'acétate de cynnamyle et
- au moins un composé choisi parmi le benzoate de benzyle, le trans-2-méthoxycinnamaldéhyde, la coumarine, l'eugénol, le safrole, le béta-caryophyllène, le linalol et le cinéole, notamment le benzoate de benzyle, le safrole, le béta-caryophyllène, le linalol et le cinéole, ici au moins le benzoate de benzyle et un composé choisi parmi le linalol, le safrole, le béta-caryophyllène et le cinéole.

Selon l'invention, la composition pour son utilisation dans le traitement ou la prévention des infections bactériennes comprend au moins les composants différents choisis chacun respectivement dans un des groupes suivants :

- trans-cinnamaldéhyde,
- acétate de cinnamyle,
- benzoate de benzyle, et
- au moins un composé choisi parmi le linalol en une teneur allant de 0,25 à 5 % en poids par rapport au poids total de la composition, le safrole, le béta-caryophyllène, et le cinéole.

Le trans-cinnamadéhyde est présent dans la composition en une teneur pouvant aller de 50 à 90 % en poids, en particulier de 50 à 85 % en poids, voire de 55 à 80 % en poids par rapport au poids total de la composition.

Le trans-2-méthoxycinnamaldéhyde peut être présent en une teneur allant de 0,5 à 15 % en poids, notamment de 1 à 10 % en poids, en particulier de 1,5 à 8 % en poids, voire de 2,5 à 7,5 % en poids par rapport au poids total de la composition.

L'acétate de cinnamyle peut être présent en une teneur allant de 0,1 à 8 % en poids, notamment de 0,25 à 5 % en poids et en particulier de 0,5 à 3 % en poids par rapport au poids total de la composition.

La coumarine peut être présente en une teneur allant de 0,25 à 5 % en poids, notamment de 0,5 à 3,5 % en poids et en particulier de 0,75 à 2,25 % en poids par rapport au poids total de la composition.

Cependant, dans un mode de réalisation particulier, la teneur en coumarine est inférieure à 1 % en poids, notamment inférieure à 0,5 % en poids, voire la composition est dépourvue de coumarine.

Le linalol peut être présent en une teneur allant de 0,25 à 5 % en poids et en particulier de 0,5 à 3 % en poids par rapport au poids total de la composition.

Le cinéole peut être présent en une teneur allant de 0,1 à 8 % en poids, notamment de 0,25 à 5 % en poids

et en particulier de 0,5 à 3 % en poids par rapport au poids total de la composition.

Le safrole peut être présent en une teneur allant de 0,01 à 5 % en poids, notamment de 0,05 à 2 % en poids et en particulier de 0,1 à 1 % en poids par rapport au poids total de la composition.

Cependant, dans un mode de réalisation particulier, la teneur en safrole est inférieure à 1 % en poids, notamment inférieure à 0,5 % en poids, voire la composition est dépourvue de safrole.

Le béta-caryophyllène peut être présent en une teneur allant de 0,1 à 5 % en poids, notamment de 0,25 à 3 % en poids et en particulier de 0,5 à 2 % en poids par rapport au poids total de la composition.

Cependant, dans un mode de réalisation particulier, la teneur en béta-caryophyllène est inférieure à 1 % en poids, notamment inférieure à 0,5 % en poids, voire la composition est dépourvue de béta-caryophyllène.

L'eugénol peut être présent en une teneur allant de 0,01 à 15 % en poids, notamment de 0,1 à 10 % en poids et en particulier de 0,2 à 7,5 % en poids par rapport au poids total de la composition.

Cependant, dans un mode de réalisation particulier, la teneur en eugénol est inférieure à 1 % en poids, notamment inférieure à 0,5 % en poids, voire la composition est dépourvue d'eugénol.

Le benzoate de benzyle peut être présent en une teneur allant de 0,01 à 3 % en poids, notamment de 0,05 à 2 % en poids et en particulier de 0,1 à 1 % en poids par rapport au poids total de la composition.

Cette composition pour son utilisation antibactérienne peut éventuellement comprendre un ou plusieurs composants additionnels.

Avantageusement la composition est dépourvue de gaiacol.

La composition pour son utilisation antibactérienne peut comprendre:

- 50 à 90 % en poids par rapport au poids total de la composition de trans-cinnamaldéhyde,
- 0,5 à 15 % en poids de trans-2-méthoxycinnamaldéhyde,
- 0,1 à 8 % en poids d'acétate de cinnamyle,
- 0,1 à 8 % en poids de linalol,
- 0,25 à 5 % en poids de coumarine, et
- 0,1 à 5 % en poids de béta-caryophyllène.

La composition pour son utilisation dans le traitement ou la prévention des infections bactériennes selon l'invention peut comprendre au moins un type d'huile essentielle issue d'écorces et/ou des feuilles, notamment de tronc, de rameaux et/ou des rameaux entiers, de plantes du genre cinnamomum, en particulier de cannelier.

Selon un mode de réalisation particulier la composition pour son utilisation antibactérienne comprend une huile essentielle de cannelle de chine et/ou une huile essentielle d'écorce de cannelle.

Dans le cadre de la présente invention, l'expression « huile essentielle » désigne aussi bien :

- l'huile essentielle naturelle, c'est-à-dire celle obtenue par extraction (le plus souvent par distillation) ; que
- l'huile essentielle purifiée dans laquelle certains composés naturellement présents sont enlevé, une telle huile purifié peut notamment être une huile rectifiée, c'est-à-dire celle obtenue par extraction (le plus souvent par distillation) puis par une étape supplémentaire (le plus souvent une distillation secondaire) destinée à enlever certains composés naturellement présents (par exemple la coumarine) ; que
- l'huile essentielle synthétique, obtenue par synthèse chimique, qui pourra contenir tout ou partie seulement des composés naturellement présents.

La cannelle de chine est également appelée cannelier, son nom latin est *Cinnamomi cassiae aetheroleum.* D'après la pharmacopée européenne, l'huile essentielle de cannelle de chine est obtenue par entraînement à la vapeur d'eau des feuilles et jeunes rameaux de *Cinnamomum cassia* Blume (*C. aromaticum* Nees). Ces composants principaux sont les suivants (pourcentages exprimés en poids par rapport au poids total de l'huile essentielle) :

- aldéhyde trans-cinnamique : 70 à 90 %
- acétate de cinnamyle : 1 à 6 %
- eugénol : < 0,5 %
- trans-2-méthoxycinnaamaldéhyde : 3 à 15 %
- coumarine : 1,5 à 4 %.

L'écorce de cannelle est également appelée cannelle dite de Ceylan, son nom latin est *Cinnamomi zeylanicii corticis aetheroleum.* D'après la pharmacopée européenne, l'huile essentielle de cannelle de Ceylan est obtenue par entraînement à la vapeur d'eau de l'écorce de jeunes tiges de *Cinnamomum zeylanicum* Nees. Ces composants principaux sont les suivants (pourcentages exprimés en poids par rapport au poids total de l'huile essentielle) :

- aldéhyde trans-cinnamique : 55 à 75 %
- eugénol : < 7,5 %
- trans-2-méthoxycinnamaldéhyde : 0,1 à 1 %
- coumarine : < 0,5 %
- cinéole : < 3,0 %
- linalol : 1 à 6 %
- β-caryophyllène : 1 à 4 %
- Safrole : < 0,5 %
- Benzoate de benzyle : < 1 %.

Les huiles essentielles sont utilisées en médecine traditionnelle depuis plus de 3000 ans. Au travers de l'histoire, cet usage a évolué jusqu'a aboutir au début du 20ème siècle à la naissance d'une branche de la phytothérapie allopathique, l'aromathérapie.

Il s'agit d'une médecine traditionnelle et empirique qui utilise les huiles essentielles pour traiter un ensemble disparate de maladies. La pharmacognosie reconnaît d'ailleurs trois actions principales aux huiles essentielles et à leurs terpènes: anti-infectieux, antispasmodique et irritantes.

Parmi ces huiles essentielles, l'huile essentielle de cannelle de chine et l'huile essentielle d'écorce de cannelle sont décrites pour leurs nombreuses propriétés bénéfiques : positivante, anti-infectieuse, antibactérienne, tonique et stimulante générale et sexuelle, respiratoire et nerveuse, hyperémiante, anesthésiante, anticoagulante.

L'activité dite « antibactérienne » de ces huiles essentielles décrite jusqu'à ce jour est principalement une activité désinfectante, mise à profit dans la conservation de denrées alimentaires. Cette activité est donc centrée sur l'utilisation dans le domaine alimentaire (préservation des aliments) de ces huiles essentielles de cannelle, ou leur principal composant actif, l'aldéhyde trans-cinnamique. Les activités de ces huiles ont été déterminée par des méthodes en général peu fiable et peu reproductibles, pouvant conduire à des résultats imprécis, voire faux.

En particulier, aucune utilisation pharmaceutique antibactérienne avec un spectre d'activité notamment vis-à-vis de bactéries résistantes et/ou de bactéries anaérobies de compositions comprenant notamment de l'aldéhyde trans-cinnamique n'a été envisagée à ce jour.

Tout particulièrement la composition selon l'invention, pour son utilisation antibactérienne pharmaceutique, est dépourvue d'antibiotiques, en particulier d'antibiotiques classiques, notamment tels qu'énoncés dans le présent texte.

Selon un autre de ses aspects, l'invention a encore pour objet une composition, notamment pharmaceutique, ou un médicament, en particulier un antibiotique, pour son utilisation dans le traitement ou la prévention des infections bactériennes, comprenant, notamment à titre d'actif antibactérien, de l'aldéhyde trans-cinnamique ou une composition antibactérienne telle que définie ci-dessus, pour le traitement ou la prophylaxie d'une maladie, notamment de ville ou nosocomiale, en particulier provoquée par une bactérie, tout particulièrement résistante aux antibactériens, en particulier ceux utilisés classiquement, par exemple chez un animal, notamment chez un mammifère, et en particulier chez un être humain.

Tout particulièrement, ladite composition, notamment pharmaceutique, ou médicament est destinée au traitement ou à la prévention d'infections bactériennes en particulier provoquées par des bactéries anaérobies.

L'actif antibactérien peut correspondre à la composition antibactérienne telle que définie ci-dessus et donc notamment comprendre, au moins des composants différents choisis chacun dans un des groupes suivants :

- trans-cinnamaldéhyde,
- au moins un composé choisi parmi l'acétate de cinnamyl, le trans-2-méthoxycinnamaldéhyde, l'eugénol et la coumarine, notamment l'acétate de cinnamyle, ici au moins l'acétate de cinnamyle, et
- au moins un composé choisi parmi le benzoate de benzyle, le trans-2-méthoxycinnamaldéhyde, la coumarine, l'eugénol, le safrole, le béta-caryophyllène, le linalol et le cinéole, notamment le benzoate de benzyle, le safrole, le béta-caryophyllène, le linalol et le cinéole, ici au moins le benzoate de benzyle et un composé choisi parmi le linalol, le safrole, le béta-caryophyllène ou le cinéole.

L'actif antibactérien peut encore comprendre en outre du cinnamaldéhyde, au moins un composé choisi parmi le trans-2-méthoxycinnamaldéhyde, l'acétate de cinnamyle, la coumarine, le linalol, le béta-caryophyllène, l'eugénol, le cinéole, le benzoate de benzyle et le safrole, voire au moins un composé choisi parmi le trans-2-méthoxycinnamaldéhyde, l'acétate de cinnamyle, la coumarine, le linalol et le béta-caryophyllène.

Tout particulièrement, l'actif comprend, voire consiste en, au moins quatre, et encore plus particulièrement au moins cinq des composés énoncé ci-dessus.

Tout particulièrement, l'actif antibactérien comprend du trans-cinnamaldéhyde, au moins un composé choisi parmi le trans-2-méthoxycinnamaldéhyde, l'acétate de cinnamyle, et la coumarine, en l'occurrence l'acétate de cinnamyle et au moins un composé choisi parmi le linalol et le béta-caryophyllène.

L'actif antibactérien peut encore comprendre ou consister en une huile essentielle de cannelle de chine, une huile essentielle d'écorce de cannelle ou un mélange de ces huiles essentielles.

Enfin, les compositions selon l'invention dites « synthétiques » ou « purifiées », c'est-à-dire comprenant un nombre de composants limités peuvent également présenter une activité améliorée, des toxicités et/ou des effets indésirables diminués.

Par ailleurs, les compositions selon l'invention peuvent présenter une activité bactéricide en présence de substance(s) interférente(s), par exemple d'albumine bovine et/ou d'érythrocytes de mouton.

Par « bactérie résistante », notamment aux antibactériens on entend, au sens de la présente invention, une bactérie résistante à au moins un, notamment au moins deux, en particulier au moins trois, voire au moins quatre, antibiotique et/ou antibactérien, ou famille d'antibiotique et/ou d'antibactérien, classiquement utilisé. L'antibiotique et/ou l'antibactérien peut être choisi parmi les composés faisant partie des grandes familles listées ci-dessous.

Par « bactérie multi-résistante » on entend, au sens de la présente invention une bactérie résistante à plusieurs antibiotique(s) et/ou antibactérien(s), en particulier pour lesquels l'espèce devrait être sensible, ou *a priori* sensible, tout particulièrement une bactérie présente au moins deux résistances non naturelles.

Selon la présente invention on entend par « antibiotique » une substance antibactérienne permettant d'aider un organisme à lutter contre une infection bactérienne.

Par « actif antibactérien », on entend selon la présente invention, toute molécule présentant des propriétés bactériostatiques ou bactéricides notamment *in vitro,* par exemple dans une composition, notamment pharmaceutique, alimentaire ou cosmétique, pour la désinfection d'espaces industriels ou d'élevages, ou *in vivo,* en particulier chez l'animal, et plus particulièrement chez l'homme, et/ou des propriétés fongicides, tout particulièrement vis-à-vis des levures.

Selon un autre de ses aspects, l'invention a pour objet l'utilisation d'une composition telle que définie dans la revendication 1 en tant qu'agent anti-bactérien ou agent de conservation, en particulier dans une composition cosmétique, pharmaceutique et/ou alimentaire. Les teneurs en agent antibactérien et/ou de conservation peuvent être du même ordre, voire identiques à celles énoncées ci-dessous.

Une substance est bactériostatique lorsqu'elle suspend ou diminue la multiplication des germes. Expérimentalement, on mesure la concentration minimale inhibitrice (CMI), qui est la plus faible concentration de substance à laquelle on n'observe plus de croissance bactérienne après 18 à 24h de contact dans des conditions favorables à la croissance bactérienne.

Une substance est bactéricide lorsqu'elle détruit définitivement la vitalité d'une bactérie. Expérimentalement, on mesure la chute logarithmique de la population microbienne. La bactéricidie se définit comme une chute de 3 Log de population bactérienne. La « bactéricidie » au sens de la présente invention peut également être définie comme dans l'exemple 7.

Selon une variante avantageuse de l'invention, l'actif antibactérien et/ou la composition, notamment pharmaceutique et/ou antibactérienne, est bactéricide.

Les antibiotiques, qui sont des antibactériens, connus et classiquement utilisés à ce jour appartiennent notamment aux grandes familles suivantes :

- aminosides,
- bêtalactamines, comme les bêtalactamines céphalosporines, les bêtalactamines pénicillines, et les autres bêtalactamines (carbapénèmes, monobactame),
- cyclines (doxycycline, limécycline, métacycline, minocycline, tétracycline, oxtétracycline, tigécycline),
- glycopeptides (téicoplanine, vancomycine) et polypeptides,
- macrolides et macrolides apparentés (lincosamides, ketolides, synergistines),
- quinolones dont fluoroquinolones,
- peptides antibactériens, notamment la gramicidine,
- phages, et
- autres (acide fusidique, noxytioline, daptomycine, fosfomycine, oxazolidinone, phénicolés, polymyxines, rifampicine, ...).

De plus en plus de bactéries deviennent résistantes à l'une ou plusieurs de ces classes d'antibactériens, notamment d'antibiotiques.

Les principaux agents antibactériens utilisés à ce jour sont plus actifs, voire sont spécifiques, vis-à-vis soit des bactéries à gram négatif soit des bactéries à gram positif. En particulier, les principaux agents antibactériens utilisés à ce jour sont plus actifs, voire sont spécifiques aux bactéries à gram positif. Il existe donc un besoin d'agents antibactériens capables d'arrêter le développement ou de détruire la vitalité des bactéries à gram négatif, et avantageusement également des bactéries à gram positif.

Or, de manière surprenante et très avantageuse, les compositions selon l'invention présentent des activités

antibactériennes tant sur les bactéries à gram positif que sur les bactéries à gram négatif. Les compositions selon l'invention peuvent être efficaces sur de telles bactéries non résistantes et/ou résistantes, voire multi-résistantes.

En particulier, il a été constaté que les compositions selon l'invention sont actives vis-à-vis des bactéries résistantes à gram négatif du genre :

- *Pseudomonas,* et plus particulièrement *P. aeruginosa ;*
- *Acinetobacter,* et plus particulièrement *A. baumanii ;*
- *Escherichia,* et plus particulièrement *E. coli ;*
- *Enterobacter,* et plus particulièrement *E. aerogenes.*

Mais il a également été constaté que l'aldéhyde trans-cinnamique et les compositions selon l'invention peuvent être actif vis-à-vis des bactéries résistantes à gram positif, en particulier celles du genre :

- *Staphylococcus* et plus particulièrement *S. aureus* et/ou
- *Enterococcus,* et plus particulièrement *E. faecalis.*

Les compositions selon l'invention peuvent être actives vis-à-vis de bactéries anaérobies, en particulier celles du genre :

- Bacteroides, en particulier *B. fragilis* et *B. thetaiotaomicron,*
- Eggerthella, en particulier *E. lenta,*
- Peptostroptococcus, en particulier *P. micros, P. spp,* et *P. anaerobius,*
- Clostridium, en particulier *C. perfringens* et *C. difficile.*
- Micromonas

Dans une première variante de l'invention, la bactérie est du genre *Pseudomonas.* Cette bactérie peut être résistante. Elle se caractérise par l'observation d'au moins une résistance suivante :

- résistance aux fluoroquinolones ;
- résistance aux céphalosporines, en particulier de $1^{ère}$, $2^{ème}$ ou $3^{ème}$ génération ;
- production d'une pénicillinase, c'est-à-dire résistance aux bêtalactamines pénicillines, notamment en cas d'hyperproduction de céphalosporinase chromosomique ;
- production d'une bétalactamase à spectre élargi (BLSE) ;
- production d'une carbapénèmase , notamment de type VIM-2 ;
- défaut de porine, en particulier de la porine D2, ce qui peut entraîner une résistance aux bêtalactamines autres que pénicillines et céphalosporines ;
- résistance aux aminosides.

Dans une deuxième variante de l'invention, la bactérie est du genre *Acinetobacter.* Cette bactérie peut être résistante. Elle peut se caractériser par l'observation d'au moins une résistance suivante :

- multirésistance ;
- bétalactamase à spectre étendu vietnamienne (Vietnamese expanded Spectrum betalactamase VEB-1).

Dans une troisième variante de l'invention, la bactérie est du genre *Escherichia.* Cette bactérie peut être résistante. Elle peut se caractériser par l'observation d'au moins une résistance suivante :

- résistance aux fluoroquinolones et aux quinolones ;
- résistance aux céphalosporines, en particulier de $1^{ère}$, $2^{ème}$ ou $3^{ème}$ génération ;
- production d'une bétalactamase à spectre élargi (BLSE) ;
- production d'une pénicillinase.

Dans une quatrième variante de l'invention, la bactérie est du genre *Staphyloccocus.* Cette bactérie peut être résistante. Elle peut se caractériser par l'observation d'au moins une résistance suivante :

- résistance à la méticilline ;
- résistance aux aminosides ; notamment résistance tobramycine/kanamycine : phénotype KT ;
- résistance aux fluoroquinolones.

Dans une cinquième variante de l'invention, la bactérie est du genre *Enteroccocus.* Cette bactérie peut être résistante. Elle peut se caractériser par l'observation d'au moins une résistance suivante :

- résistance aux aminosides ;
- résistance aux macrolides et macrolides apparentés.

Dans une sixième variante de l'invention, la bactérie est du genre *Enterobacter.* Cette bactérie peut être résistante. Elle peut se caractériser par l'observation d'au moins une résistance de production d'une bétalactamase à spectre élargi (BLSE).

Selon un mode de réalisation particulier, l'invention a pour objet une composition pharmaceutique, ou un médicament, comprenant, ou consistant en, une composition antibactérienne selon l'invention, à titre d'actif antibactérien.

Cette composition pharmaceutique, ou ce médicament, peut être destinée au traitement ou à la prévention des infections bactériennes, en particulier provoquée par une bactérie résistante aux antibactériens, par exemple chez un animal, notamment chez un mammifère, et en particulier chez l'homme.

Tout particulièrement, elle est destinée au traitement ou à la prévention d'infections bactériennes provoquées par des bactéries anaérobies

La composition, notamment pharmaceutique, ou le médicament peut être destinée au traitement ou à la prophylaxie de maladies, notamment nosocomiales, liées à des infections fongiques, notamment par des levures, et/ou bactériennes, notamment par des bactéries résistantes, voire multi-résistantes.

Ces maladies peuvent être choisies dans le groupe constitué par les infections de l'appareil urinaire, les infections du système respiratoire, les infections de l'appareil digestif, les infections du système nerveux central, les infections de la peau et des tissus mous, les infections des os, articulations et muscles, les infections du système vasculaire, le choc septique, le pied diabétique, et les escarres.

Ces bactéries résistantes se rencontrent de plus en plus fréquemment, en particulier en milieu hospitalier, et sont à l'origine de nombreux syndromes nosocomiaux. Lutter contre ces bactéries et/ou ces levures, permet donc de prévenir ou traiter de nombreux syndromes nosocomiaux.

Selon une variante avantageuse de l'invention, la composition selon l'invention est destinée au traitement ou à la prophylaxie de maladies nosocomiales, notamment via des levures et/ou des bactéries, notamment par des bactéries résistantes, voire multi-résistantes.

Lesdites maladies peuvent être choisies dans le groupe constitué par les infections de l'appareil urinaire, les infections du système respiratoire, les infections de l'appareil digestif, les infections du système nerveux central, les infections de la peau et des tissus mous, les infections des os, articulations et muscles, les infections du système vasculaire, le choc septique, le pied diabétique, et les escarres.

Par « infection nosocomiale » on entend au sens de la présente invention toute infection microbienne, virale et/ou fongique contractée en milieu hospitalier, notamment avec une apparition des symptômes au moins 24h, voire au moins 48h, après l'admission du patient.

La présente invention porte également sur le traitement et la prévention des syndromes rencontrés dans le cadre des étiologies suivantes :

- infections sans passage des barrières épithéliales, telles que les infections par inhalation ou par ingestion ;
- infections avec passage des barrières épithéliales, telles que les infections par piqûres, coupures, blessures, transplantation, transfusion ;
- infections liées à l'utilisation d'un dispositif médical invasif (prothèse, stent) ;
- infections opératoires, péri opératoires, postopératoires ;
- infections consécutives de brûlures.

La composition selon l'invention peut également être destinée au traitement et/ou à la prévention du portage de bactéries, en particulier de staphylocoques dorés, dans les différentes flores du patient, notamment flore cutanée, flore buccale et flore nasopharyngée.

Les maladies induites par la prolifération de bactéries, et plus particulièrement les maladies nosocomiales, se rencontrent plus souvent chez des personnes dites sensibles. La composition selon l'invention est donc toute particulièrement destinée au traitement et/ou à la prophylaxie des maladies nosocomiales du/chez une personne immunodéprimée ou immunodéficiente. Les personnes immunodéprimées ou immunodéficientes peuvent être les personnes âgées (plus de 65 ans), les nourrissons (moins de 12 mois), les enfants en bas âge (moins de 4 ans), les personnes suivant une corticothérapie et les personnes ayant bénéficié d'une transplantation et les personnes atteintes du SIDA.

Dans la composition pharmaceutique ou le médicament, on utilise, à titre d'actif antibactérien, la composition antibactérienne telle que définie précédemment.

Le trans-cinnamaldéhyde peut provenir de l'huile essentielle de cannelle de chine ou l'huile essentielle d'écorce

de cannelle, et éventuellement d'une association de ces deux huiles essentielles.

La composition pharmaceutique peut comprendre, ou consister en, la composition antibactérienne. En particulier la composition pharmaceutique peut comprendre la composition antibactérienne à titre de principe actif et notamment antibactérien.

Ainsi, la composition selon l'invention, peut comprendre, voire consister en, une huile essentielle de cannelle de chine et/ou une huile essentielle d'écorce de cannelle, elles peuvent présenter un ratio massique allant de 9:1 à 1 :9, notamment allant de 4:1 à 1 :4, tout particulièrement allant de 2 :1 à 1 :2, voire un ratio de 1 :1.

Les résultats expérimentaux montrent, pour l'huile essentielle de cannelle de chine, une activité antibactérienne sur des souches résistantes *Pseudomonas aeruginosa*, *E. coli, Staphyllococcus aureus* et d'entérocoques à des concentrations volumiques variant de 0,125 à 1 % (v/v). A plus faible concentration (0,06 %), on observe toujours une activité antibactérienne sur toutes les souches résistantes testées. A concentration encore plus faible (0,03 %), on observe toujours une activité antibactérienne sur une majorité des souches résistantes *Pseudomonas aeruginosa*, *E. coli, Staphyllococcus aureus, Enterococcus faecium, Acinetobacter aerogenes, Acinetobacter baumanii.*

Les résultats expérimentaux montrent, pour l'huile essentielle d'écorce de cannelle, une activité antibactérienne sur des souches résistantes *Pseudomonas aeruginosa*, *Escherichia coli, Staphyllococcus aureus, Acinetobacter aerogenes* et d'entérocoques à des concentrations volumiques variant de 0,125 à 1 % (v/v). A plus faible concentration (0,06 %), on observe toujours une activité antibactérienne sur toutes les souches résistantes *E. coli, Staphyllococcus aureus* et d'entérocoques. A concentration encore plus faible (0,03 %), l'activité antibactérienne n'est observée que sur deux souches résistantes *Staphyllococcus aureus.*

On constate donc que ces deux huiles essentielles conduisent à l'obtention de très bons résultats sur des souches bactériennes résistantes, prélevées en milieu hospitalier ; les résultats les meilleurs étant obtenus avec l'huile essentielle de cannelle de chine.

Un effet de synergie, inattendu, a également été constaté en particulier pour l'association huile essentielle de cannelle de chine et huile essentielle d'écorce de cannelle. Lorsqu'on utilise une telle association (dans un ratio volumique 1 :1), l'activité antibactérienne est observée sur presque toutes les souches même à une concentration très faible de 0,06 % (v/v).

L'invention a donc également pour objet l'utilisation, en synergie, d'une huile essentielle de cannelle de chine associée à une huile essentielle d'écorce de cannelle. L'association peut comprendre les deux huiles essentielles à toute hauteur, le ratio massique 1 :1 étant préféré.

C'est la première fois qu'un tel effet de synergie entre ces deux huiles est observé. L'invention s'étend donc à toute composition pharmaceutique comprenant à titre d'actif antibactérien une association d'huile essentielle de cannelle de chine et d'huile essentielle d'écorce de cannelle, de préférence dans un ratio volumique de 1 :1. La composition pharmaceutique, comprenant une association d'huile essentielle de cannelle de chine et d'huile essentielle d'écorce de cannelle, pourra être utilisée, dans le cadre de l'invention, en tant que médicament antibactérien : tant à l'encontre des bactéries non résistantes que des bactéries résistantes décrites auparavant. Lesdites bactéries peuvent être des bactéries à gram positif ou à gram négatif, en particulier des bactéries, résistantes ou non, à gram négatif du genre :

- *Pseudomonas,* et plus particulièrement *Pseudomonas aeruginosa ;*
- *Acinetobacter,* et plus particulièrement *Acinetobacter baumanii ;*
- *Escherichia,* et plus particulièrement *Escherichia coli ;*
- *Enterobacter,* et plus particulièrement *Enterobacter aerogenes ;*

ou des bactéries à gram positif, résistantes ou non, du genre :

- *Staphylococcus* et plus particulièrement *Staphylococcus aureus ;*
- *Enterococcus,* et plus particulièrement *Enterococcus faecalis.*

La « synergie » évoquée dans le présent texte peut notamment être calculé de la manière suivante :

$$FIC_{index} = (CMI_{A/B} / CMI_A) + (CMI_{B/A} / CMI_B)$$

Où

- $FIC_{index}$ est l'index de concentration inhibitrice fractionnelle (« Fractionnal Inhibitory Concentration),
- $CMI_A$ = CMI du composé A, en particulier de l'antibiotique A, seul,
- $CMI_B$ = CMI du composé B, en particulier de l'antibiotique B, seul,
- $CMI_{A/B}$ = CMI du composé A, en particulier de l'antibiotique A, dans le mélange A+B,

- $CMI_{B/A}$= CMI du composé B, en particulier de l'antibiotique B, dans le mélange A+B,

Cette formule permet de déterminer si il y a un effet de synergie, d'addition, indifférent, ou d'antagonisme de la manière suivante, lorsque :

- $FIC_{index}$ est inférieur ou égal à 0,5 il y a un effet de synergie,
- $FIC_{index}$ est supérieur à 0,5 et inférieur ou égal à 1 il y un effet d'addition,
- $FIC_{index}$ est supérieur à 1 et inférieur ou égal à 2 il y un effet indifférent, et
- $FIC_{index}$ est supérieur à 2 il y un effet antagoniste.

Selon un autre de ses aspects, l'invention décrit une composition dans laquelle la composition antibactérienne est diluée, cette composition diluée pouvant comprendre de 1 à 90 % en poids, notamment de 5 à 75 % en poids, en particulier de 10 à 50 % en poids, en particulier de 20 à 50 % en poids par rapport au poids total de la composition diluée de la composition antibactérienne.

Selon un mode de réalisation particulier la composition, antibactérienne pharmaceutique, ou le médicament présente une teneur en trans-cinnamaldéhyde allant de 0,1 à 6,5 % en poids, notamment de 0,2 à 5,5 % en poids, en particulier de 0,4 à 5 % en poids, voire de 0,5 à 4,5 % en poids par rapport au poids total de la composition.

Par ailleurs, la teneur en actif antibactérien, correspondant à la composition antibactérienne telle que définie ci-dessus, peut aller de 0,05 à 7,5 % en poids, notamment de 0,1 à 6 % en poids, en particulier de 0,2 à 5 % en poids, voire de 0,5 à 5 % en poids, et tout particulièrement de 0,75 à 5 % en poids par rapport au poids total de la composition.

La composition peut être destinée à être appliquée sur la peau et/ou les muqueuses, sur un dispositif destiné à entrer en contact avec la peau et/ou les muqueuses et/ou sur un dispositif destiné à rompre la barrière épithéliale.

Un des objet de la présente invention est encore un procédé de prévention ou de prophylaxie des infections bactériennes et/ou fongiques, notamment nosocomiale, comprenant une étape d'application d'une composition selon l'invention sur la surface cutanée et/ou la muqueuse dont l'épithélium est destiné à être rompu ou lésé, par exemple par un cathéter, notamment vasculaire ou urinaire, ou présentant une brèche permettant le passage de bactéries et/ou de levures.

La composition, et en particulier pharmaceutique, peut être formulée de toute manière appropriée pour son utilisation finale.

Elle peut en particulier être formulée sous une forme la rendant propre à une administration par voie locale, par voie orale (per os), par voie rectale, par voie pulmonaire, par intraveineuse, par voie sous-cutanée, par voie cutanée, par voie intramusculaire et/ou par voie intrapéritonéale. Elle peut à ce titre comprendre tout excipient approprié.

La composition peut tout particulièrement être formulée sous une forme adaptée à ces administrations, et en particulier à une administration topique ou par injection.

La composition peut notamment se présenter sous forme de liquide, d'émulsion, de crème, de gel. En général elle se présente sous toute forme permettant une application aisée sur la surface, par exemple la peau, la muqueuse, ou sur le dispositif traité.

Avantageusement, et en particulier pour les formes topiques et/ou à usage local, par exemple cutanée, elles peuvent se présenter sous une forme permettant à la composition de ne pas s'écouler trop facilement de manière à rester sur le support sur lequel elles sont appliquées.

Tout particulièrement dans le cas d'une utilisation préventive, la composition, en particulier pharmaceutique, peut se présenter sous une forme permettant une application topique, comme une crème, une pommade, une poudre, un gel ou une émulsion. Avantageusement la composition présente un écoulement sur elle-même faible ou nul, notamment à 37°C, en particulier afin qu'elle reste en place à l'endroit souhaité.

La composition, en particulier pharmaceutique, peut tout particulièrement se présenter sous forme hydrophobe, c'est-à-dire dépourvue d'eau et/ou de solvant polaire.

La composition, en particulier lorsqu'elle se présente sous forme de gel, peut présenter une viscosité allant de 500 à 2 000, en particulier de 750 à 1 500 centipoise (cP). La viscosité peut être mesurée à 25°C avec un mobile CPE52 à 250 tr/mn.

La composition, en particulier pharmaceutique, notamment sous forme de pommade, de gel ou de crème, peut comprendre du polyéthylène glycol, notamment du macrogol 400, des triglycérides à chaînes moyennes et/ou de l'huile de soja, en particulier la composition comprend un seul de ces composés.

La composition, en particulier pharmaceutique, peut comprendre une teneur totale en ces composés allant de 70 à 99 %, notamment de 90 à 97 % en poids par rapport au poids total de la composition

Cette composition peut en outre comprendre de la silice colloïdale, notamment à une teneur allant de 2 à 20 % en poids, en particulier allant de 3 à 10 % en poids, voire d'environ 5 % en poids par rapport au poids total de la composition.

La composition, en particulier pharmaceutique, peut également être une émulsion, en particulier cette émulsion

comprend un gélifiant.

Le gélifiant peut être un polymère, notamment de polyvinyl pyrrolidone, en particulier de la povidone, par exemple de la povidone K30®. La teneur en gélifiant peut aller de 40 à 60 % en poids par rapport au poids total de la composition.

L'émulsion peut comprendre un tensioactif, par exemple de type polysorbate, polyglyceryl oléate ou glycérides d'acides capriques/caprylique. La teneur en tensioactif peut aller de 1 à 5 % en poids par rapport au poids total de la composition.

L'émulsion peut comprendre de l'eau, notamment en une teneur allant de 40 à 60 % en poids par rapport au poids total de la composition.

La composition, peut également être formulée dans le but de pouvoir être appliquée, en particulier dans un but de traitement de surface, sur des dispositifs médicaux, invasifs ou non, des dispositifs médicaux actifs implantables, comme des prothèses ou des stents, et des dispositifs implantables. Elle peut à ce titre comprendre tout excipient approprié.

Selon un mode de réalisation particulier, la composition, notamment antibactérienne ou pharmaceutique comprend également un antiseptique à large spectre, comme de la chlorhexidine ou de l'iode, en particulier de la polyvidone iodée, par exemple la bétadine.

Selon un mode de réalisation particulier, l'invention décrit un pansement ou un patch comprenant une composition, notamment antibactérienne ou pharmaceutique. Un tel pansement ou patch est en particulier destiné à la prévention des maladies nosocomiales, en particulier dans le cas d'utilisation de cathéter.

En particulier ledit patch ou pansement est conditionné de manière stérile.

Selon un autre mode de réalisation, en particulier dans le cas d'un traitement préventif ou d'un traitement de surface, la composition, notamment antibactérienne ou pharmaceutique, est une crème, une émulsion, une pommade, une poudre ou un gel.

Selon encore un autre mode de réalisation, en particulier dans le cas d'un traitement curatif, la composition, notamment antibactérienne ou pharmaceutique, est formulé de manière à pouvoir être injectée, en particulier par intraveineuse, par voie sous-cutanée, par voie trans-cutanée, par voie intramusculaire et/ou par voie intrapéritonéale. La composition peut alors être sous forme d'une composition hydrophile ou hydrophobe.

Une composition hydrophile peut comprendre un gélifiant par exemple tel que défini ci-dessus, et/ou un tensioactif, notamment tel que défini ci-dessus.

Une composition hydrophobe peut comprendre du polyéthylène glycol, notamment du macrogol 400, des triglycérides à chaînes moyennes et/ou de l'huile de soja, éventuellement dilué dans de l'eau.

Selon encore un autre de ses aspects, l'invention décrit l'utilisation de trans-cinnamaldéhyde, notamment de trans-cinnamaldéhyde et d'au moins un composé choisi parmi le trans-2-méthoxycinnamaldéhyde, l'acétate de cinnamyle, la coumarine, le linalol, le béta-caryophyllène, l'eugénol, le cinéole, le benzoate de benzyle et le safrole, voire au moins un composé choisi parmi le trans-2-méthoxycinnamaldéhyde, l'acétate de cinnamyle, la coumarine, le linalol et le béta-caryophyllène, voire d'une composition selon l'invention, en tant qu'actif antibactérien.

Cette utilisation peut être *in vivo* ou *ex vivo.*

Selon encore un autre de ses aspects, l'invention décrit l'utilisation de trans-cinnamaldéhyde, notamment de trans-cinnamaldéhyde et d'au moins un composé choisi parmi le trans-2-méthoxycinnamaldéhyde, l'acétate de cinnamyle, la coumarine, le linalol, le béta-caryophyllène, l'eugénol, le cinéole, le benzoate de benzyle et le safrole, voire au moins un composé choisi parmi le trans-2-méthoxycinnamaldéhyde, l'acétate de cinnamyle, la coumarine, le linalol et le béta-caryophyllène, voire d'une composition selon l'invention, pour la préparation d'un actif antibactérien.

Tout particulièrement, l'actif antibactérien comprend, voire consiste en, au moins quatre, et encore plus particulièrement au moins cinq des composés énoncé ci-dessus.

Selon un mode de réalisation particulier, l'actif antibactérien correspond à la composition antibactérienne, en particulier à l'un ou à l'autre mode de réalisation spécifique décrit ci-dessus.

Selon un autre de ses aspects, l'invention décrit l'utilisation de trans-cinnamaldéhyde, notamment de cinnamaldéhyde et d'au moins un composé choisi parmi le trans-2-méthoxycinnamaldéhyde, l'acétate de cinnamyle, la coumarine, le linalol, le béta-caryophyllène, l'eugénol, le cinéole, le benzoate de benzyle et le safrole, voire au moins un composé choisi parmi le trans-2-méthoxycinnamaldéhyde, l'acétate de cinnamyle, la coumarine, le linalol et le béta-caryophyllène, voire d'une composition selon l'invention, pour la préparation d'un médicament destiné au traitement ou à la prophylaxie d'une maladie nosocomiale provoquée par une bactérie résistante aux antibactériens chez un animal, notamment un mammifère, et en particulier chez un être humain.

Selon un autre de ses aspects, l'invention à pour objet un procédé de préparation d'une surface corporelle, notamment de la peau, d'une muqueuse, ou d'une surface, par exemple une extrémité, d'un dispositif, comme un cathéter, comprenant au moins une étape consistant à recouvrir cette surface par une composition telle que définie précédemment.

Selon un mode de réalisation particulier, ce procédé n'est pas effectué sur la surface corporelle d'un animal

et notamment d'un être humain.

Ce procédé peut permettre de limiter les risques d'infections bactériennes, et en particulier d'infections nosocomiales.

Dans le cas où une composition est appliquée sur la peau, elle peut permettre, dans le cas où la barrière cutanée est rompue de limiter, voire d'empêcher, que des bactéries, en particulier résistantes, ne viennent infecter l'organisme.

Bien entendu, les différentes caractéristiques de l'invention sont susceptibles d'être combinées entre elles.

Les exemples qui suivent illustrent l'invention et ne sont pas limitatifs.

**Exemple 1** : **protocole de test en gélose**

Pour une bonne accessibilité des bactéries (multiplication uniquement en phase aqueuse) aux huiles essentielles, il est indispensable de trouver une méthode de solubilisation en phase aqueuse. Cette étape est négligée dans beaucoup de travaux qui utilisent directement la phase lipidique inaccessible aux bactéries (disque imbibé d'huile posé sur une gélose). De plus pour un usage topique des huiles cette phase de solubilisation est également indispensable et dans le but d'une application pratique, les surfactants et adjuvants de solubilisation sont choisis aussi pour leur bonne tolérance cutanée.

La solubilisation fait appel à des tensioactifs (Tween 20, Tween 80) et à des adjuvants de solubilisation (propylène glycol, glycérol, mannitol, éthanol, amidon modifié). Cependant, l'utilisation d'éthanol nous semble inappropriée (antibactérien, hautement volatil). Les essais préliminaires avec un mélange de Tween 80 et propylène glycol à 20 % ont donné des solubilisations suffisantes pour la plupart des huiles (10 g d'huile essentielle, 29 g de Tween 80, 61 g de propylène glycol à 20 % molaire). Avant les premières analyses, nous avons testé le tensioactif et l'adjuvant de solubilisation dans les proportions indiquées pour vérifier l'absence d'activité antibactérienne. Une souche d'*E. coli* a été inhibée par ce mélange et nous avons donc dû réduire la concentration en propylène glycol à 10 % molaire, à cette concentration aucune souche n'est inhibée.

La préparation finale comprend : 1,0 ml d'huile essentielle, 3,4 ml de Tween 80 et 5,6 ml de solution aqueuse de propylène glycol à 10 % molaire.

**Exemple 2 : souches bactériennes**

Les souches testées sont isolées de divers prélèvements humains (sang, urines, aspirations pulmonaires, etc..). Elles ont été isolées chez des patients non-infectés à l'entrée à l'hôpital et chez lesquels une infection est survenue après au moins 48h d'hospitalisation.
Les souches étudiées dans le présent exemple sont les suivantes :

- *E. coli* ATCC 25922, pour le contrôle qualité (tableau 1),
- Souches de *Pseudomonas aeruginosa* : souches 8128, 8129, 8132, 8134 (tableau 2), et 9007 (présentées sous le tableau 6),
- Souches de E. coli: souches 8154, 8155, 8156 et 8157 (tableau 3), et 9003 (tableau 6)
- Souches de staphylocoques dorés *(S. aureus)* : souches 8147, 8239 et 8240 (tableau 4),
- Souches d'entérocoques : 8152 et 8153 (tableau 5),
- Souche Enterococcus faecium (9001) (tableau 6),
- Souche Enterobacter aerogenes (9004) (tableau 6),
- Souche Acinetobacter baumanii (9010) (présentées sous le tableau 6).

Trois catégories cliniques ont été retenues pour l'interprétation des tests de sensibilité *in vitro* : Sensible (S), Résistant (R) et Intermédiaire (I).

- Les souches catégorisées S sont celles pour lesquelles la probabilité de succès thérapeutique est forte dans le cas d'un traitement par voie systémique avec la posologie recommandée dans le résumé des caractéristiques du produit (RCP), rédigé par l'Agence Française de Sécurité Sanitaire des Produits de Santé (AFSSAPS).
- Les souches catégorisées R sont celles pour lesquelles il existe une forte probabilité d'échec thérapeutique quels que soient le type de traitement et la dose d'antibiotique utilisée.
- Les souches catégorisées I sont celles pour lesquelles le succès thérapeutique est imprévisible. Ces souches forment un ensemble hétérogène pour lequel les résultats obtenus *in vitro* ne sont pas prédictifs d'un succès thérapeutique. En effet, ces souches :

    - peuvent présenter un mécanisme de résistance dont l'expression *in vitro* est faible, avec pour conséquence

leur classement dans la catégorie S. Cependant, *in vivo,* une partie de ces souches apparaît résistante au traitement ;

- peuvent présenter un mécanisme de résistance dont l'expression n'est pas suffisante pour justifier un classement dans la catégorie R, mais suffisamment faible pour espérer un effet thérapeutique dans certaines conditions (fortes concentrations locales ou posologies accrues) ;

La catégorie intermédiaire est aussi une zone tampon qui tient compte des incertitudes techniques et biologiques.

Méthodologie

La sensibilité des souches aux antibiotiques a été déterminée par une technique de diffusion selon les recommandations 2008 du CA-SFM (Comité de l'antibiogramme de la société française de microbiologie).

*Etablissement des valeurs critiques délimitant les catégories cliniques*

Les valeurs des concentrations et des diamètres critiques définies pour chaque antibiotique sont établies en tenant compte de plusieurs paramètres :

- la distribution des concentrations minimales inhibitrices (CMI) pour des populations de souches définies et appartenant à chacune des espèces bactériennes impliquées en pathologie humaine ;
- les concentrations humorales et tissulaires qui sont obtenues avec les posologies recommandées dans le résumé des caractéristiques du produit (RCP) ;
- la confrontation des résultats obtenus in vitro et des résultats obtenus in vivo (essais cliniques) ;
- la variabilité statistique des méthodes utilisées pour mesurer les CMI et les diamètres des zones d'inhibition.

Ainsi sont définies deux concentrations critiques : la concentration critique basse c et la concentration critique haute C auxquelles correspondent des diamètres critiques D, et d, respectivement.

*Procédure et critères de catégorisation des souches*

Aux regards des concentrations et des diamètres critiques sont considérées comme :

- sensibles (S), les souches pour lesquelles la CMI de l'antibiotique testé est inférieure ou égale à la concentration critique basse (c), ce qui équivaut à un diamètre supérieur ou égal au diamètre critique D ;
- résistantes (R), les souches vis-à-vis desquelles la CMI de l'antibiotique testé est supérieure à la concentration critique haute C, correspondant à un diamètre inférieur au diamètre critique d;
- de sensibilité intermédiaire (I), les souches vis-à-vis desquelles la CMI de l'antibiotique testé et du diamètre correspondant sont compris entre les deux concentrations critiques et les deux diamètres critiques.

Les essais ont été réalisés sur un milieu Mueller Hinton. Un inoculum ajusté à l'échelle McFarland 0,5 est ensuite dilué au 1/100. Les boîtes de Petri sont ensemencées par écouvillonnage. La lecture est réalisée après 24h d'incubation à 37°C.
Les résultats bruts sont ensuite interprétés selon les règles de lecture interprétative du CA-SFM. Deux exemples, toute souche de staphylocoque résistante à la méticilline est résistante à l'ensemble des β-lactamines (bétalactamines), de même en cas de résistance à la gentamicine, le staphylocoque doré est résistant obligatoirement à la Kanamycine (et amikacine) et à la tobramycine.
Le contrôle de qualité de la méthode de diffusion est réalisé à l'aide de la souche de *E. coli* ATCC 25922.

Résultats de l'antibiogramme :

- Contrôle de qualité (tableau 1)

Tableau 1 : contrôle de qualité

| ***E. coli* ATCC 25922** | | |
|---|---|---|
| Antibiotiques | Diamètre (D) | Catégorisation (Cat.) |
| **PENICILLINES** | | |
| Amoxicilline | 23 | S |
| Amoxicilline + acide clavulanique | 22 | S |
| Ticarcilline | 25 | S |
| **CEPHALOSPORINES** | | |
| Céfalotine | 20 | S |
| Ceftazidime | 25 | S |
| Céfépime | 25 | S |
| Céfoxitine | 24 | S |
| **AMINOSIDES** | | |
| Kanamycine | 22 | S |
| Gentamicine | 25 | S |
| Tobramycine | 24 | S |
| **CARBAPENEMES** | | |
| Imipenem | 34 | S |
| **QUINOLONES** | | |
| Acide nalidixique | 28 | S |
| **FLUOROQUINOLONES** | | |
| Lévofloxacine | 31 | S |

Pour la souche du contrôle de qualité, les diamètres sont conformes.

- Souches de *Pseudomonas aeruginosa* (tableau 2)

Tableau 2 : souches 8128, 8129, 8132 et 8134

| | 8128 | | 8129 | | 8132 | | 8134 | |
|---|---|---|---|---|---|---|---|---|
| Antibiotiques | D | Cat. | D | Cat. | D | Cat. | D | Cat. |
| **PENICILLINES** | | | | | | | | |
| Amoxicilline | 0 | R | 0 | R | 0 | R | 0 | R |
| Amoxicilline+ acide clavulanique | 0 | R | 0 | R | 0 | R | 0 | R |
| Ticarcilline | 21 | I | 13 | R | 12 | R | 23 | S |
| **CEPHALOSPORINES** | | | | | | | | |
| Céfalotine | 0 | R | 0 | R | 0 | R | 0 | R |
| Ceftazidime | 25 | S | 0 | R | 26 | S | 25 | S |
| Céfépime | 30 | S | 27 | S | 25 | S | 29 | S |
| Céfoxitine | 0 | R | 0 | R | 0 | R | 0 | R |
| **AMINOSIDES** | | | | | | | | |
| Kanamycine | 9 | R | 10 | R | 0 | R | 14 | R |
| Gentamicine | 27 | S | 21 | S | 16 | R | 0 | R |

(suite)

| | | | | | | | | |
|---|---|---|---|---|---|---|---|---|
| **AMINOSIDES** | | | | | | | | |
| Tobramycine | 30 | S | 27 | S | 0 | R | 11 | R |
| **CARBAPENEMES** | | | | | | | | |
| Imipenem | 18 | I | 36 | S | 13 | R | 26 | S |
| **QUINOLONES** | | | | | | | | |
| Acide nalidixique | 0 | R | 0 | R | 0 | R | 0 | R |
| **FLUOROQUINOLONES** | | | | | | | | |
| Lévofloxacine | 23 | S | 26 | S | 0 | R | 0 | R |

On constate que :

- la souche 8128 est multi-résistante. Elle est aussi résistante aux pénicillines et aux quinolones.
- la souche 8129 est hyperproductrice de céphalosporinase chromosomique. Elle est aussi résistante aux pénicillines et aux quinolones.
- la souche 8132 est multi-résistante dont défaut de porine D2.
- la souche 8134 est résistante aux quinolones, aux fluoroquinolones et aux aminosides.

▪ Souches de E. coli (tableau 3)

Tableau 3: souches 8154, 8155, 8156 et 8157

| | 8154 | | 8155 | | 8156 | | 8157 | |
|---|---|---|---|---|---|---|---|---|
| Antibiotiques | D | Cat. | D | Cat. | D | Cat. | D | Cat. |
| **PENICILLINES** | | | | | | | | |
| Amoxicilline | 0 | R | 24 | S | 25 | S | 0 | R |
| Amoxicilline+ acide clavulanique | 14 | S | 24 | S | 22 | S | 30 | S |
| Ticarcilline | 0 | R | 31 | S | 28 | S | 0 | R |
| **CEPHALOSPORINES** | | | | | | | | |
| Céfalotine | 21 | S | 21 | S | 20 | S | 19 | S |
| Ceftazidime | 30 | S | 30 | S | 33 | S | 30 | S |
| Céfépime | 32 | S | 35 | S | 32 | S | 32 | S |
| Céfoxitine | 26 | S | 25 | S | 26 | S | 30 | S |
| **AMINOSIDES** | | | | | | | | |
| Kanamycine | 24 | S | 23 | S | 27 | S | 24 | S |
| Gentamicine | 21 | S | 21 | S | 25 | S | 25 | S |
| Tobramycine | 24 | S | 24 | S | 22 | S | 24 | S |
| **CARBAPENEMES** | | | | | | | | |
| Imipenem | 33 | S | 34 | S | 34 | S | 20 | I |
| **QUINOLONES** | | | | | | | | |
| Acide nalidixique | 24 | S | 28 | S | 27 | S | 0 | R |
| **FLUOROQUINOLONES** | | | | | | | | |
| Lévofloxacine | 36 | S | 31 | S | 31 | S | 11 | R |

On constate que :

- La souche 8154 est une souche productrice de pénicillinase
- La souche 8155 est une souche non résistante
- La souche 8156 est une souche non résistante
- La souche 8157 est une souche productrice de pénicillinase et résistante aux fluoroquinolones.

▪ Souches de staphylocoques dorés *(S. aureus)* (tableau 4)

Tableau 4: souches 8147, 8239 et 8240

| | 8147 | | 8239 | | 8240 | |
|---|---|---|---|---|---|---|
| Antibiotiques | D | Cat. | D | Cat. | D | Cat. |
| **PENICILLINES** | | | | | | |
| Amoxicilline | 26 | R | 12 | R | 17 | R |
| Amoxicilline+ acide clavulanique | 25 | R | 27 | R | 25 | R |
| **CEPHALOSPORINES** | | | | | | |
| Céfalotine | 17 | R | 17 | R | 17 | R |
| **AMINOSIDES** | | | | | | |
| Kanamycine | 22 | S | 26 | S | 12 | R |
| Gentamicine | 26 | S | 28 | S | 27 | S |
| Tobramycine | 30 | S | 31 | S | 0 | R |
| **FLUOROQUINOLONES** | | | | | | |
| Lévofloxacine | 10 | R | 10 | R | 0 | R |
| **LINCOSAMIDES** | | | | | | |
| Clindamycine | 29 | S | 31 | S | 30 | S |
| **STREPTOGRAMINES** | | | | | | |
| Pristinamycine | 29 | S | 32 | S | 27 | S |

On constate que :

- La souche 8147 est une souche méticilline sensible.
- La souche 8239 est une souche méticilline résistante.
- La souche 8240 est une souche méticilline résistante et fluoroquinolone résistante.

▪ Souches d’entérocoques (tableau 5)

Tableau 5 : Souches 8152 et 8153

| | 8152 | | 8153 | |
|---|---|---|---|---|
| Antibiotiques | Diamètre | Catégorisation | Diamètre | Catégorisation |
| **PENICILLINES** | | | | |
| Amoxicilline | 32 | S | 33 | S |
| Amoxicilline + acide clavulanique | 30 | S | 32 | S |

(suite)

| | | | | |
|---|---|---|---|---|
| **CEPHALOSPORINES** | | | | |
| Céfoxitine | 0 | R | 25 | S |
| **AMINOSIDES** | | | | |
| Kanamycine | 10 | R | 14 | R |
| Gentamicine | 15 | R | 14 | R |
| Tobramycine | 11 | R | 14 | R |
| **FLUOROQUINOLONES** | | | | |
| Lévofloxacine | 29 | S | 26 | S |
| **LINCOSAMIDE** | | | | |
| Clindamycine | 0 | R | 0 | R |
| **STREPTOGRAMINES** | | | | |
| Pristinamycine | 23 | S | 22 | S |

Les deux souches présentent une résistance naturelle et une résistance aux aminosides (bas niveau). Elles sont en outre résistantes MLSb (résistance à érythromycine et clindamycine et sensibilité pristinamycine).

- Souches sélectionnées pour leurs multirésistances (tableau 6)

Tableau 6 : Souches 9001, 9003 et 9004

| 9001 Enterobacter faecium | | 9003 E. Coli | | 9004 Enterobacter aerogenes | |
|---|---|---|---|---|---|
| Pen G | nl | Amox | R | Amox | R |
| Ampi | R | Augmentin | S | Augmentin | R |
| Kana HC | R | Ticar | R | Ticar | R |
| GentaHC | S | Claventin | R | Claventin | R |
| Strepto HC | R | Pipera-tazo | S | Pipera-tazo | S |
| Tetra | S | Cefalotine | R | Cefalotine | R |
| Erythro | R | Cefoxatime | R | Cefoxatime | R |
| Clinda 2 | R | Cefepime | R | Cefepime | R |
| Dalf-quinu | I | Ceftazidime | R | Ceftazidime | R |
| Linezolide | S | Imipenem | S | Imipenem | S |
| Levoflo | R | Eratpeneme | S | Eratpeneme | S |
| Moxiflo | R | Gentamicine | S | Gentamicine | S |
| Trim+Sulf | R | Tobramycine | S | Tobramycine | R |
| Teico | S | Amikacine | S | Amikacine | I |
| Vanco | R | Colimycine | S | Colimycine | S |
| Furanes | S | Bactrim | S | Bactrim | R |
| Chloram | S | Noroxine | R | Noroxine | R |
| | | Ciprofloxacine | R | Ciprofloxacine | R |

Ont aussi été testées deux souches génétiquement définies :

- la souche 9007, Pseudomonas aeruginosa à carbapénémase type VIM-2, tableau 7, et

- la souche 9010, Acinetobacter baumanii, à BLSE type VEB-1, tableau 7.

Tableau 7 : Souche 9007 et 9010

| | 9007 Pseudomonas aeruginosa | 9010 Acinetobacter |
|---|---|---|
| AMOXICILLINE | R | R |
| AMOXICILLINE-clavu | R | R |
| CÉFALOTINE | R | R |
| TICARCILLINE | R | R |
| CEFTAZIDIME | S | R |
| CÉFÉPIME | S | R |
| CÉFOXITINE | R | R |
| KANAMYCINE | R | R |
| GENTAMICINE | S | R |
| TOBRAMYCINE | S | R |
| IMIPENEM | R | S |
| ACIDE NALIDIXIQUE | R | R |
| LÉVOFLOXACINE | S | R |

**Exemple 3 (exemple comparatif): Trans-cinnamaldéhyde**

Matériels :

- Souches bactériennes (celles décrites à l'exemple 2)
- Bouillon coeur cervelle (BH)
- Gélose Mueller Hinton (en tube incliné ou en tubes de 18 et 19 mL) (MHA)
- Diluant Ringer cystéiné
- Tubes Falcon pour dilution
- Tubes stériles
- Trans-cinnamaldéhyde

Protocole :

La veille, repiquer les souches dans un milieu BH et en gélose inclinée MHA. Préparer les tubes de milieu MHA de 19 ml et 18 ml. Préparer des tubes de 5 ml de propylène glycol à 10 % molaire pour les différentes dilutions.

Le jour même, réaliser la solubilisation du trans-cinnamaldéhyde :

1 ml de trans-cinnamaldéhyde
3,4 ml de Tween 80
5,6 ml de propylène glycol à 10% molaire.

Préparer le mélange Tween 80 et propylène glycol à 10 % molaire à l'avance. Mettre 9 ml de ce mélange dans des tubes Falcon et ajouter 1 ml d'huile.

Lorsque le trans-cinnamaldéhyde est solubilisé, réaliser les dilutions successives au ½ dans le propylène glycol à 10 % molaire.

Ajouter 1 ml des dilutions adéquates dans les tubes contenant 19 ml de milieu, bien homogénéiser et verser dans la boite de Petri. Pour la première dilution (10% volumique de trans-cinnamaldéhyde), préparer un tube de 18 mL de MHA et 2 mL de trans-cinnamaldéhyde à 10% volumique pour augmenter la plus forte concentration à tester (1%

volumique).

Préparation de l'inoculum :

Ajouter 10 ml de RC sur la gélose inclinée qui contient la souche puis reprendre 1 goutte de cette suspension et la distribuer dans un tube de 10 ml de RC, on obtient une suspension à $10^6$ UFC/ ml. Mettre 1 ml de cette suspension dans la cupule du Steers. (les 33 souches sont ensemencées sur la même boite). Ensemencer chacune des boites et incuber 24 h à 37 °C.

Des boites témoins (MHA seule) sont également faites pour vérifier la croissance de toutes les souches et qu'il n'y a pas de problème de contamination.

Résultats :

Les résultats obtenus avec différentes souches sont reportés dans le tableau 7 suivant :

Tableau 8 : activité antibactérienne du trans-cinnamaldéhyde

| Référence | Nom | 1 | 0,5 | 0,25 | 0,125 | 0,06 | 0,03 | 0,015 | 0.0075 | 0,0038 |
|---|---|---|---|---|---|---|---|---|---|---|
| ***ATCC 25922*** | *E.coli* | - | - | - | - | - | - | - | + | + |
| *8128* | *Pseudomonas* | - | - | - | - | - | + | + | + | + |
| *8129* | *Pseudomonas* | - | - | - | - | - | + | + | + | + |
| *8132* | *Pseudomonas* | - | - | - | - | + | + | + | + | + |
| *8134* | *Pseudomonas* | - | - | - | - | - | - | - | + | + |
| *8154* | *E.coli* | - | - | - | - | - | - | + | + | + |
| *8155* | *E.coli* | - | - | - | - | - | - | + | + | + |
| *8156* | *E.coli* | - | - | - | - | - | - | + | + | + |
| *8157* | *E.coli* | - | - | - | - | - | - | + | + | + |
| *8147* | *Staphylococcus* | - | - | - | - | - | - | + | + | + |
| *8239* | *Staphylococcus* | - | - | - | - | - | - | + | + | + |
| *8240* | *Staphylococcus* | - | - | - | - | - | - | + | + | + |
| *8152* | *Enterococcus* | - | - | - | - | - | - | + | + | + |
| *8153* | *Enterococcus* | - | - | - | - | - | - | + | + | + |
| *9001* | *Enterococcus* | - | - | - | - | - | - | + | + | + |
| *9003* | *E.coli* | - | - | - | - | - | + | + | + | + |
| *9004* | *Enterobacter* | - | - | - | - | - | + | + | + | + |
| *9007* | *PS aeruginosa* | - | - | - | - | - | + | + | + | + |
| *9010* | *A baumanii* | - | - | - | - | - | - | - | + | + |

On constate que le trans-cinnamaldéhyde présente une activité antibactérienne tant sur les bactéries à gram positif que sur les bactéries à gram positif. Cet effet est également observé sur des souches résistantes. Cet effet est en outre observé pour des concentrations volumiques faibles.

L'incertitude est de ± une dilution.

**Exemple 4 (exemple comparatif): Huile essentielle de cannelle de Chine**

L'exemple 3 est répété mais le trans-cinnamaldéhyde est remplacé par une huile essentielle naturelle d'écorce de cannelle.

Résultats :

Les résultats obtenus avec différentes souches sont reportés dans le tableau 8 suivant :

Tableau 9 : activité antibactérienne de l'huile essentielle de Cannelle de chine

| Référence | Nom | 1 | 0,5 | 0,25 | 0,125 | 0,06 | 0,03 |
|---|---|---|---|---|---|---|---|
| ***ATCC 25922*** | *E.coli* | - | - | - | - | - | - |
| *8128* | *Pseudomonas* | - | - | - | - | + | + |
| *8129* | *Pseudomonas* | - | - | - | - | + | + |
| *8132* | *Pseudomonas* | - | - | - | + | + | + |
| *8134* | *Pseudomonas* | - | - | - | - | + | + |
| *8154* | *E.coli* | - | - | - | - | - | + |
| *8155* | *E.coli* | - | - | - | - | - | + |
| *8156* | *E.coli* | - | - | - | - | - | + |
| *8157* | *E.coli* | - | - | - | - | - | + |
| *8147* | *Staphylococcus* | - | - | - | - | - | + |
| *8239* | *Staphylococcus* | - | - | - | - | - | + |
| *8240* | *Staphylococcus* | - | - | - | - | - | + |
| *8152* | *Enterococcus* | - | - | - | - | - | + |
| *8153* | *Enterococcus* | - | - | - | - | - | + |
| *9001* | *Enterococcus* | - | - | - | - | - | - |
| *9003* | *E.coli* | - | - | - | - | - | - |
| *9004* | *Enterobacter* | - | - | - | - | - | + |
| *9007* | *PS aeruginosa* | - | - | - | - | + | + |
| *9010* | *A baumanii* | - | - | - | - | - | - |

On constate que l'huile essentielle de Cannelle de Chine présente une activité antibactérienne tant sur les bactéries à gram positif que sur les bactéries à gram positif. Cet effet est également observé sur des souches résistantes. Cet effet est en outre observé pour des concentrations volumiques faibles.

L'incertitude est de ± une dilution.

**Exemple 5 (exemple comparatif): Huile essentielle d'écorce de cannelle**

L'exemple 3 est répété mais le trans-cinnamaldéhyde est remplacé par une huile essentielle naturelle d'écorce de cannelle.

Résultats :

Les résultats obtenus avec différentes souches sont reportés dans le tableau 9 suivant :

Tableau 10 : activité antibactérienne de l'huile essentielle d'écorce de Cannelle

| Référence | Nom | 1 | 0,5 | 0,25 | 0,125 | 0,06 | 0,03 | 0,015 | 0,0075 | 0,0004 |
|---|---|---|---|---|---|---|---|---|---|---|
| ***ATCC 25922*** | *E.coli* | - | - | - | - | - | + | + | + | + |
| *8128* | *Pseudomonas* | - | - | - | - | + | + | + | + | + |
| *8129* | *Pseudomonas* | - | - | - | - | + | + | + | + | + |
| *8132* | *Pseudomonas* | - | - | - | + | + | + | + | + | + |

(suite)

| Référence | Nom | 1 | 0,5 | 0,25 | 0,125 | 0,06 | 0,03 | 0,015 | 0,0075 | 0,0004 |
|---|---|---|---|---|---|---|---|---|---|---|
| *8134* | *Pseudomonas* | - | - | - | - | + | + | + | + | + |
| *8154* | *E.coli* | - | - | - | - | - | + | + | + | + |
| *8155* | *E.coli* | - | - | - | - | - | + | + | + | + |
| *8156* | *E.coli* | - | - | - | - | - | + | + | + | + |
| *8157* | *E.coli* | - | - | - | - | - | + | + | + | + |
| *8147* | *Staphylococcus* | - | - | - | - | - | + | + | + | + |
| *8239* | *Staphylococcus* | - | - | - | - | - | + | + | + | + |
| *8240* | *Staphylococcus* | - | - | - | - | - | + | + | + | + |
| *8152* | *Enterococcus* | - | - | - | - | - | + | + | + | + |
| *8153* | *Enterococcus* | - | - | - | - | - | + | + | + | + |
| *9001* | *Enterococcus* | - | - | - | - | - | + | + | + | + |
| *9003* | *E.coli* | - | - | - | - | - | + | + | + | + |
| *9004* | *Enterobacter* | - | - | - | - | + | + | + | + | + |
| *9007* | *PS aeruginosa* | - | - | - | - | + | + | + | + | + |
| *9010* | *A baumanii* | - | - | - | - | - | + | + | + | + |

On constate que l'huile essentielle d'écorce de Cannelle présente une activité antibactérienne tant sur les bactéries à gram positif que sur les bactéries à gram positif. Cet effet est également observé sur des souches résistantes. Cet effet est en outre observé pour des concentrations volumiques faibles.

L'incertitude est de ± une dilution.

**Exemple 6 : Associations d'huile essentielle de cannelle de chine et d'huile essentielle d'écorce de cannelle**

L'exemple 3 est répété mais le trans-cinnamaldéhyde est remplacé par un mélange 1 :1 (volumique), 1 :2 (volumique) ou 2 :1 (volumique) d'une huile essentielle naturelle de cannelle de chine et d'une huile essentielle naturelle d'écorce de cannelle.

Dans le cas d'un mélange 1 :1 de deux huiles cela signifie qu'il y a 1% de chaque huile et dans le cas d'un mélange 2 :1 la première huile est présente à 1,3 % et la deuxième à 0,7%. Ces pourcentages sont des % en volume par rapport au volume total.

Résultats :

Les résultats obtenus avec différentes souches sont reportés dans les tableaux 10 à 12 suivants :

Tableau 11 : activité antibactérienne de l'association huile essentielle d'écorce de Cannelle et huile essentielle de cannelle de chine (1 : 1)

| Référence | Nom | 1 | 0,5 | 0,25 | 0,125 | 0,06 | 0,03 | 0,015 | 0,0075 | 0,0038 |
|---|---|---|---|---|---|---|---|---|---|---|
| ***ATCC 25922*** | *E.coli* | - | - | - | - | - | - | - | + | + |
| *8128* | *Pseudomonas* | - | - | - | - | - | - | + | + | + |
| *8129* | *Pseudomonas* | - | - | - | - | - | - | + | + | + |
| *8132* | *Pseudomonas* | - | - | - | - | - | + | + | + | + |
| *8134* | *Pseudomonas* | - | - | - | - | - | - | + | + | + |
| *8154* | *E.coli* | - | - | - | - | - | - | - | + | + |
| *8155* | *E.coli* | - | - | - | - | - | - | - | + | + |

(suite)

| Référence | Nom | 1 | 0,5 | 0,25 | 0,125 | 0,06 | 0,03 | 0,015 | 0,0075 | 0,0038 |
|---|---|---|---|---|---|---|---|---|---|---|
| *8156* | *E.coli* | - | - | - | - | - | - | - | + | + |
| *8157* | *E.coli* | - | - | - | - | - | - | - | + | + |
| *8147* | *Staphylococcus* | - | - | - | - | - | - | - | + | + |
| *8239* | *Staphylococcus* | - | - | - | - | - | - | - | + | + |
| *8240* | *Staphylococcus* | - | - | - | - | - | - | - | + | + |
| *8152* | *Enterococcus* | - | - | - | - | - | - | - | + | + |
| *8153* | *Enterococcus* | - | - | - | - | - | - | - | - | + |
| *9001* | *Enterococcus* | - | - | - | - | - | - | - | - | + |
| *9003* | *E. coli* | - | - | - | - | - | - | - | - | + |
| *9004* | *Enterobacter* | - | - | - | - | - | - | - | + | + |
| *9007* | *PS aeruginosa* | - | - | - | - | - | - | - | - | + |
| *9010* | *A baumanii* | - | - | - | - | - | - | - | - | - |

Tableau 12 : activité antibactérienne de l'association huile essentielle d'écorce de Cannelle et huile essentielle de cannelle de chine (2 : 1)

| Référence | Nom | 1 | 0,5 | 0,25 | 0,125 | 0,06 | 0,03 | 0,015 | 0,0075 | 0,0038 |
|---|---|---|---|---|---|---|---|---|---|---|
| ***ATCC 25922*** | *E.coli* | - | - | - | - | - | - | - | + | + |
| *8128* | *Pseudomonas* | - | - | - | - | - | + | + | + | + |
| *8129* | *Pseudomonas* | - | - | - | - | - | + | + | + | + |
| *8132* | *Pseudomonas* | - | - | - | - | + | + | + | + | + |
| *8134* | *Pseudomonas* | - | - | - | - | - | + | + | + | + |
| *8154* | *E.coli* | - | - | - | - | - | - | + | + | + |
| *8155* | *E.coli* | - | - | - | - | - | - | + | + | + |
| *8156* | *E.coli* | - | - | - | - | - | - | - | + | + |
| *8157* | *E.coli* | - | - | - | - | - | - | - | + | + |
| *8147* | *Staphylococcus* | - | - | - | - | - | - | + | + | + |
| *8239* | *Staphylococcus* | - | - | - | - | - | - | + | + | + |
| *8240* | *Staphylococcus* | - | - | - | - | - | - | + | + | + |
| *8152* | *Enterococcus* | - | - | - | - | - | + | + | + | + |
| *8153* | *Enterococcus* | - | - | - | - | - | + | + | + | + |

Tableau 13 : activité antibactérienne de l'association huile essentielle d'écorce de Cannelle et huile essentielle de cannelle de chine (1 :2)

| Référence | Nom | 1 | 0,5 | 0,25 | 0,125 | 0,06 | 0,03 | 0,015 | 0,0075 | 0,0038 |
|---|---|---|---|---|---|---|---|---|---|---|
| ***ATCC 25922*** | *E.coli* | - | - | - | - | - | + | + | + | + |
| *8128* | *Pseudomonas* | - | - | - | - | + | + | + | + | + |
| *8129* | *Pseudomonas* | - | - | - | - | + | + | + | + | + |
| *8132* | *Pseudomonas* | - | - | - | + | + | + | + | + | + |

(suite)

| Référence | Nom | 1 | 0,5 | 0,25 | 0,125 | 0,06 | 0,03 | 0,015 | 0,0075 | 0,0038 |
|---|---|---|---|---|---|---|---|---|---|---|
| *8134* | *Pseudomonas* | - | - | - | - | + | + | + | + | + |
| *8154* | *E.coli* | - | - | - | - | - | + | + | + | + |
| *8155* | *E.coli* | - | - | - | - | - | + | + | + | + |
| *8156* | *E.coli* | - | - | - | - | - | + | + | + | + |
| *8157* | *E.coli* | - | - | - | - | - | + | + | + | + |
| *8147* | *Staphylococcus* | - | - | - | - | - | + | + | + | + |
| *8239* | *Staphylococcus* | - | - | - | - | - | + | + | + | + |
| *8240* | *Staphylococcus* | - | - | - | - | - | + | + | + | + |
| *8152* | *Enterococcus* | - | - | - | - | - | + | + | + | + |
| *8153* | *Enterococcus* | - | - | - | - | - | + | + | + | + |

On constate que l'association huile essentielle d'écorce de Cannelle et huile essentielle de cannelle de chine présente une activité antibactérienne tant sur les bactéries à gram positif que sur les bactéries à gram positif. Cet effet est également observé sur des souches résistantes. Cet effet est en outre observé pour des concentrations volumiques faibles, encore plus faibles que celles observées pour chacune des huiles essentielles seules. Les résultats les meilleurs sont obtenus avec le ratio volumique 1 : 1.
L'incertitude est de ± une dilution.

**Exemple 7 : Bactéricidie**

Préparation de la suspension d'huile essentielle

On prépare un mélange contenant 3,4 ml de Tween 80 et 5,6 ml de propylène glycol et l'on ajoute dans ce mélange 1 ml d'huile (concentration finale 10%).

Préparation de l'inoculum et du mélange huile/bactéries

Après culture de 24 h en bouillon Mueller Hinton, on ajuste l'inoculum à environ $10^8$ UFC/ml ce qui correspond à une turbidité comparable à l'étalon 0,5 de la gamme de McFarland. Par dilution au dixième, on prépare ensuite un tube contenant 9 ml d'une suspension bactérienne à $10^7$ UFC/ml dans du Ringer cystéiné auquel on ajoutera soit 1 ml d'eau distillée (témoin) soit 1 ml de la suspension d'huile à 10% (soit une concentration finale à 1% d'huile).

Dénombrement des bactéries

Les dénombrements sont faits par dilutions successives au dixième des échantillons. On étale 100 µl de chacune des dilutions sur une gélose Mueller Hinton. Le dénombrement s'effectue sur la boîte de Peri qui contient entre 15 et 150 colonies. Le seuil de dénombrement est donc de 150 UFC/ml.

Pouvoir neutralisant du D/E

Pour arrêter l'activité de l'huile après un temps de contact défini, on prélève 100 µl du mélange huile + bactérie que l'on dilue dans 900 µl de diluant neutralisant de façon à bloquer l'action antibactérienne de l'huile.
Le neutralisant utilisé est le « Neutralizing broth for neutralizing and testing disinfectants and antiseptics » de Dey et Engley commercialisé chez Criterion dont la formule est :
Glucose (10 g), lécithine (7 g), peptone de caséine (5 g), Tween 80 (5 g), thiosulfate de sodium (6 g), phosphate bipotassique (3,3 g), bisulfite de sodium (2,5 g), extrait de levure (2,5 g), thioglycollate de sodium (1 g), phosphate monopotassique (0,1 g) et pourpre de bromocrésol (20 mg). La poudre obtenue est dissoute dans un litre d'eau désionisée, puis après chauffage et dissolution, le milieu est stérilisé par passage à l'autoclave à 121°C pendant 15 minutes. Le pH final est de 7,6 ± 0,2.
Un témoin de neutralisant est réalisé ainsi :

Un mélange huile + neutralisant à partie égale est mis en contact de l'inoculum bactérien. Après incubation 48 h à 37°C, le dénombrement bactérien doit ne pas être inférieur à 50% du dénombrement témoin. Cela permet de démontrer l'absence d'activité antibactérienne du neutralisant et surtout le fait que le neutralisant bloque totalement l'action antibactérienne de l'huile évitant par là le phénomène de carry-over.

Détermination de l'inoculum de départ

On réalise un dénombrement à $T_0$. On prélève 100 $\mu$l de l'inoculum de départ que l'on place dans 900 $\mu$l de diluant. Ensuite 100 $\mu$l de chacune des 6 dilutions au dixième ultérieures sont déposées sur une gélose Mueller Hinton. Après incubation des boîtes de Peri pendant 48 h à 37°C, on compte les colonies sur la gélose comme indiqué précédemment.

Mesure de l'activité bactéricide des huiles essentielles

Après 15, 30, 45 et 60 minutes de contact huile + bactéries, on prélève 100 $\mu$l du mélange produit/bactéries que l'on place dans 900 $\mu$l de neutralisant puis on réalise 2 dilutions au dixième et centième en Ringer cystéiné. On étale ensuite 100 $\mu$l de chacune des dilutions sur une gélose Mueller Hinton. Les boîtes sont ensuite incubées 48 h et les survivants sont ainsi dénombrés.

Expression des résultats

Par définition la bactéricidie est obtenue si l'on observe au minimum une chute de 3 logarithmes de l'inoculum de départ. Une courbe de bactéricidie liant le nombre de bactéries au temps de contact avec l'huile peut être tracée ; elle permet de voir si la cinétique de bactéricidie est intense et rapide ou non.

Résultats :

La composition comprenant un ratio 1 : 1 d'huile essentielle de cannelle de chine et d'huile essentielle d'écorce de cannelle (telle que décrite dans l'exemple 6) présente une bactéricidie vis-à-vis des souches 8132, 8154, 8152, 8155, ATCC 25922 (témoin), 9004 et 9001 identifiées plus précisément dans les exemples ci-dessus.
Cette composition comporte aussi une activité bactéricide sur les souches suivantes, caractérisées par la méthode décrite ci dessus :

- Staphylococcus aureus 8148 : souche résistante à la méthicilline et aux Fluoroquinolones
- Staphylococcus aureus 8237 : souche sauvage.

**Exemple 8 : Gel antibactérien**

1 ml d'un mélange 1 : 1 d'huile essentielle de cannelle de Chine et d'huile essentielle de cannelle de Ceylan est solubilisé dans 93 ml de Macrogol 400 (Lutreol 400). Puis de la silice colloïdale, 6 g est ajoutée.
L'ensemble est homogénéisé afin d'obtenir un gel non coulant présentant une viscosité (T48) de 798 cP à 25°C, mobile CPE 52, à 250 tr/mn.
Ce gel peut notamment être utilisé en prévention d'infections bactériennes et/ou fongiques, par exemple dans le cas de l'utilisation d'un cathéter.
Ledit cathéter peut être recouvert par ledit gel ou la surface présentant une brèche cutanée ou la muqueuse susceptible d'être lésée peut être recouverte dudit gel.
Un tel gel permet notamment la diminution de la teneur en bactérie d'une forme topique.

**Exemple 9 : traitement de souris infectées**

Le test est effectué sur des souris Balb/c de 10 à 12 semaines.
8 souris sont infectées par injection inrapéritonéale de $4.10^7$ *Staphyococcus aureus,* résistant à la méthicilline en phase de croissance.
Le lendemain, un traitement quotidien de 100mg/kg/j (en une administration) est engagé avec les produits suivants :
- Absence de traitement
- HE de Cannelle de Ceylan
- HE de cannelle de Ceylan + HE de cannelle de Chine (1 : 1)

Tableau 15 : Survie des souris après inoculation de MRSA

| **Souris survivantes** | Jour 1 | Jour 2 | Jour 3 | Jour 4 |
|---|---|---|---|---|
| Absence de traitement | 8/8 | 7/8 | 7/8 | 4/8 |
| Mélange Cannelle de Ceylan - Cannelle Chine (1 : 1) | 8/8 | 8/8 | 8/8 | 8/8 |
| Cannelle de Ceylan | 8/8 | 8/8 | 8/8 | 8/8 |

Il est par ailleurs observé que les souris ont un poids moyen supérieur dans le groupe Mélange Cannelle de Ceylan - Cannelle Chine, ce qui tend à montrer que les souris traitées par le mélange d'HE soient mieux traitées que celles traitées par la cannelle de Ceylan seule.
Cet exemple démontre que l'efficacité in vivo correspond à l'efficacité in vitro.

## Revendications

**1.** Composition pour son utilisation dans le traitement ou la prévention des infections bactériennes, ladite composition comprenant au moins les composants différents choisis chacun respectivement dans un des groupes suivants :

- trans-cinnamaldéhyde en une teneur allant de 50 à 90% en poids par rapport au poids total de la composition,
- acétate de cinnamyle,
- benzoate de benzyle, et
- au moins un composé choisi parmi le linalol en une teneur allant de 0,25 à 5 % en poids par rapport au poids total de la composition, le safrole, le béta-caryophyllène, et le cinéole.

**2.** Composition pour son utilisation selon la revendication 1, **caractérisée en ce qu'**elle comprend du trans-2-méthoxycinnamaldéhyde, en particulier en une teneur allant de 0,5 à 15 % en poids, de préférence de 1,5 à 8 % en poids par rapport au poids total de la composition.

**3.** Composition pour son utilisation selon l'une quelconque des revendications 1 à 2, **caractérisée en ce qu'**elle comprend de l'acétate de cinnamyle en une teneur allant de 0,1 à 8 % en poids, notamment de 0,25 à 5 % en poids, voire de 0,5 à 3 % en poids par rapport au poids total de la composition.

**4.** Composition pour son utilisation selon l'une quelconque des revendications 1 à 3, **caractérisée en ce qu'**elle comprend de la coumarine, en particulier en une teneur allant de 0,25 à 5 % en poids, notamment de 0,5 à 3,5 % en poids, voire de 0,75 à 2,25 % en poids par rapport au poids total de la composition.

**5.** Composition pour son utilisation selon l'une quelconque des revendications 1 à 4, **caractérisée en ce qu'**elle comprend du linalol, en une teneur allant de 0,5 à 3 % en poids par rapport au poids total de la composition.

**6.** Composition pour son utilisation selon l'une quelconque des revendications 1 à 5, **caractérisée en ce qu'**elle comprend du béta-caryophyllène, en particulier en une teneur allant de 0,1 à 5 % en poids, notamment de 0,25 à 3 % en poids, voire de 0,5 à 2 % en poids par rapport au poids total de la composition.

**7.** Composition pour son utilisation selon l'une quelconque des revendications 1 à 6, **caractérisée en ce qu'**elle comprend du benzoate de benzyle en une teneur allant de 0,01 à 3 %, en poids par rapport au poids total de la composition.

**8.** Composition pour son utilisation selon l'une quelconque des revendications 1 à 7, **caractérisée en ce qu'**elle comprend du cinéole en une teneur allant de 0,1 à 8 % en poids par rapport au poids total de la composition.

**9.** Composition pour son utilisation selon l'une quelconque des revendications 1 à 8, **caractérisée en ce qu'**elle comprend du safrole en une teneur allant de 0,01 à 5 % en poids par rapport au poids total de la composition.

**10.** Composition pour son utilisation selon l'une quelconque des revendications 1 à 9, **caractérisée en ce qu'**elle comprend de l'eugénol en une teneur allant de 0,01 à 15 % en poids par rapport au poids total de la composition.

**11.** Composition pour son utilisation selon l'une quelconque des revendications 1 à 10, **caractérisée en ce qu'**elle comprend une huile essentielle de cannelle de chine et/ou une huile essentielle d'écorce de cannelle, en particulier en un ratio allant de 9:1 à 1:9, notamment allant de 4:1 à 1:4, tout particulièrement allant de 2:1 à 1:2, voire un ratio de 1:1.

**12.** Composition pour son utilisation selon l'une quelconque des revendications 1 à 11, **caractérisée en ce qu'**elle est destinée au traitement ou à la prévention des infections bactériennes provoquées par une bactérie anaérobie.

**13.** Composition pour son utilisation selon la revendication 12, **caractérisée en ce qu'**elle est destinée au traitement ou à la prophylaxie d'une maladie nosocomiale, en particulier provoquée par une bactérie résistante aux antibactériens par exemple chez un animal, notamment chez un mammifère, en particulier chez l'homme.

**14.** Composition pour son utilisation selon la revendication 12 ou 13, **caractérisée en ce que** la maladie nosocomiale est choisie dans le groupe constitué par les infections de l'appareil urinaire, les infections du système respiratoire, les infections de l'appareil digestif, les infections du système nerveux central, les infections de la peau et des tissus mous, les infections des os, articulations et muscles, les infections du système vasculaire, le choc septique, le pied diabétique, et les escarres.

**15.** Composition pour son utilisation selon l'une quelconque des revendications 12 à 14, **caractérisée en ce qu'**elle est destinée au traitement ou à la prophylaxie du/chez une personne immunodéprimée ou immunodéficiente, en particulier les personnes âgés, les nourrissons, les enfants en bas âge, les personnes suivant une corticothérapie, les personnes ayant bénéficié d'une transplantation et les personnes atteintes du SIDA.

**16.** Utilisation d'une composition telle que définie selon l'une quelconque des revendications 1 à 11, en tant qu'agent de conservation.

**17.** Procédé de préparation d'un dispositif, comme un cathéter, comprenant au moins une étape consistant à recouvrir cette surface par une composition telle que définie selon l'une quelconque des revendications 1 à 11.

## Patentansprüche

**1.** Zusammensetzung zur Verwendung bei der Behandlung oder Vorbeugung von bakteriellen Infektionen, wobei die Zusammensetzung zumindest die verschiedenen Komponenten umfasst, die jeweils aus einer der folgenden Gruppen ausgewählt sind:

- trans-Cinnamaldehyd mit einem Gehalt von 50 bis 90 Gew.-% bezogen auf das Gesamtgewicht der Zusammensetzung,
- Cinnamylacetat,
- Benzylbenzoat und
- zumindest eine Verbindung, die unter Linalol mit einem Gehalt von 0,25 bis 5 Gew.-% bezogen auf das Gesamtgewicht der Zusammensetzung, Safrol, Beta-Caryophyllen und Cineol ausgewählt ist.

**2.** Zusammensetzung zur Verwendung nach Anspruch 1, **dadurch gekennzeichnet, dass** sie trans-2-Methoxycinnamaldehyd insbesondere mit einem Gehalt von 0,5 bis 15 Gew.-%, vorzugsweise 1,5 bis 8 Gew.-% bezogen auf das Gesamtgewicht der Zusammensetzung enthält.

**3.** Zusammensetzung zur Verwendung nach einem beliebigen der vorstehenden Ansprüche 1 bis 2, **dadurch gekennzeichnet, dass** sie Cinnamylacetat mit einem Gehalt von 0,1 bis 8 Gew.-%, insbesondere 0,25 bis 5 Gew.-%, im Speziellen 0,5 bis 3 Gew.-% bezogen auf das Gesamtgewicht der Zusammensetzung enthält.

**4.** Zusammensetzung zur Verwendung nach einem beliebigen der vorstehenden Ansprüche 1 bis 3, **dadurch gekennzeichnet, dass** sie Kumarin, insbesondere mit einem Gehalt von 0,25 bis 5 Gew.-%, im Speziellen 0,5 bis 3,5 Gew.-%, oder sogar von 0,75 bis 2,25 Gew.-% bezogen auf das Gesamtgewicht der Zusammensetzung enthält.

**5.** Zusammensetzung zur Verwendung nach einem beliebigen der vorstehenden Ansprüche 1 bis 4, **dadurch gekennzeichnet, dass** sie Linalol mit einem Gehalt von 0,5 bis 3 Gew.-% bezogen auf das Gesamtgewicht der Zusammensetzung enthält.

6. Zusammensetzung zur Verwendung nach einem beliebigen der vorstehenden Ansprüche 1 bis 5, **dadurch gekennzeichnet, dass** sie Beta-Caryophyllen insbesondere mit einem Gehalt von 0,1 bis 5 Gew.-%, im Speziellen 0,25 bis 3 Gew.-%, oder sogar 0,5 bis 2 Gew.-% bezogen auf das Gesamtgewicht der Zusammensetzung enthält.

7. Zusammensetzung zur Verwendung nach einem beliebigen der vorstehenden Ansprüche 1 bis 6, **dadurch gekennzeichnet, dass** sie Benzylbenzoat mit einem Gehalt von 0,01 bis 3 Gew.-% bezogen auf das Gesamtgewicht der Zusammensetzung enthält.

8. Zusammensetzung zur Verwendung nach einem beliebigen der vorstehenden Ansprüche 1 bis 7, **dadurch gekennzeichnet, dass** sie Cineol mit einem Gehalt von 0,1 bis 8 Gew.-% bezogen auf das Gesamtgewicht der Zusammensetzung enthält.

9. Zusammensetzung zur Verwendung nach einem beliebigen der vorstehenden Ansprüche 1 bis 8, **dadurch gekennzeichnet, dass** sie Safrol mit einem Gehalt von 0,01 bis 5 Gew.-% bezogen auf das Gesamtgewicht der Zusammensetzung enthält.

10. Zusammensetzung zur Verwendung nach einem beliebigen der vorstehenden Ansprüche 1 bis 9, **dadurch gekennzeichnet, dass** sie Eugenol mit einem Gehalt von 0,01 bis 15 Gew.-% bezogen auf das Gesamtgewicht der Zusammensetzung enthält.

11. Zusammensetzung zur Verwendung nach einem beliebigen der vorstehenden Ansprüche 1 bis 10, **dadurch gekennzeichnet, dass** sie ein ätherisches China-Zimtöl und/oder ein ätherisches Zimtrindenöl insbesondere in einem Verhältnis von 9:1 bis 1:9, im Speziellen von 4:1 bis 1:4, insbesondere von 2:1 bis 1:2, oder sogar in einem Verhältnis von 1:1 enthält.

12. Zusammensetzung zur Verwendung nach einem beliebigen der vorstehenden Ansprüche 1 bis 11, **dadurch gekennzeichnet, dass** sie zur Behandlung oder Vorbeugung von durch ein anaerobes Bakterium verursachten bakteriellen Infektionen bestimmt ist.

13. Zusammensetzung zur Verwendung nach Anspruch 12, **dadurch gekennzeichnet, dass** sie zur Behandlung oder Prophylaxe einer nosokomialen Erkrankung, die insbesondere durch ein gegen antibakterielle Mittel resistentes Bakterium verursacht wurde, beispielsweise bei einem Tier, insbesondere bei einem Säugetier, im Speziellen beim Menschen bestimmt ist.

14. Zusammensetzung zur Verwendung nach Anspruch 12 oder 13, **dadurch gekennzeichnet, dass** die nosokomiale Erkrankung aus der Gruppe bestehend aus Harnwegsinfektionen, Atemwegsinfektionen, Infektionen des Verdauungstrakts, Infektionen des Zentralnervensystems, Haut- und Weichgewebeinfektionen, Knochen-, Gelenk- und Muskelinfektionen, Gefäßinfektionen, septischem Schock, dem diabetischen Fuß und Druckstellen ausgewählt ist.

15. Zusammensetzung zur Verwendung nach einem beliebigen der vorstehenden Ansprüche 12 bis 14, **dadurch gekennzeichnet, dass** sie zur Behandlung oder Prophylaxe einer abwehrgeschwächten oder immundefizienten Person, insbesondere bei älteren Menschen, Säuglingen, Kleinkindern, Personen, die sich einer Kortikoidtherapie unterziehen, Personen mit einer Transplantation und Personen, die an AIDS leiden, bestimmt ist.

16. Verwendung einer Zusammensetzung, wie sie nach einem beliebigen der vorstehenden Ansprüche 1 bis 11 definiert ist, als Konservierungsmittel.

17. Verfahren zur Herstellung einer Vorrichtung wie eines Katheters, umfassend zumindest einen Schritt mit Beschichten der Oberfläche mit einer Zusammensetzung, wie sie nach einem beliebigen der vorstehenden Ansprüche 1 bis 11 definiert ist.

## Claims

1. Composition for use in the treatment or prevention of bacterial infections, said composition including at least the different components each selected from one of the following groups, respectively:

   - trans-cinnamaldehyde in a proportion ranging from 50% to 90% by weight relative to the total weight of the

composition,
- cinnamyl acetate,
- benzyl benzoate, and
- at least one compound selected from linalool in a proportion ranging from 0.25% to 5% by weight relative to the total weight of the composition, safrole, beta-caryophyllene, and cineole.

**2.** Composition for use according to claim 1, **characterized in that** it includes *trans*-*2*-methoxycinnamaldehyde, in particular in a proportion ranging from 0.5% to 15% by weight, preferably from 1.5% to 8% by weight relative to the total weight of the composition.

**3.** Composition for use according to any one of claims 1 to 2, **characterized in that** it includes cinnamyl acetate in a proportion ranging from 0.1% to 8% by weight, notably from 0.25% to 5% by weight, or even from 0.5% to 3% by weight relative to the total weight of the composition.

**4.** Composition for use according to any one of claims 1 to 3, **characterized in that** it includes coumarin, in particular in a proportion ranging from 0.25% to 5% by weight, notably from 0.5% to 3.5% by weight, or even from 0.75% to 2.25% by weight relative to the total weight of the composition.

**5.** Composition for use according to any one of claims 1 to 4, **characterized in that** it includes linalool, in a proportion ranging from 0.5% to 3% by weight relative to the total weight of the composition.

**6.** Composition for use according to any one of claims 1 to 5, **characterized in that** it includes beta-caryophyllene, in particular in a proportion ranging from 0.1% to 5% by weight, notably from 0.25% to 3% by weight, or even from 0.5% to 2% by weight relative to the total weight of the composition.

**7.** Composition for use according to any one of claims 1 to 6, **characterized in that** it includes benzyl benzoate in a proportion ranging from 0.01% to 3%, by weight relative to the total weight of the composition.

**8.** Composition for use according to any one of claims 1 to 7, **characterized in that** it includes cineole in a proportion ranging from 0.1% to 8% by weight relative to the total weight of the composition.

**9.** Composition for use according to any one of claims 1 to 8, **characterized in that** it includes safrole in a proportion ranging from 0.01% to 5% by weight relative to the total weight of the composition.

**10.** Composition for use according to any one of claims 1 to 9, **characterized in that** it includes eugenol in a proportion ranging from 0.01% to 15% by weight relative to the total weight of the composition.

**11.** Composition for use according to any one of claims 1 to 10, **characterized in that** it includes essential oil of Chinese cinnamon and/or essential oil of cinnamon bark, in particular in a ratio ranging from 9:1 to 1:9, notably ranging from 4:1 to 1:4, particularly ranging from 2:1 to 1:2, or even a ratio of 1:1.

**12.** Composition for use according to any one of claims 1 to 11, **characterized in that** it is intended for the treatment or prevention of bacterial infections caused by an anaerobic bacterium.

**13.** Composition for use according to claim 12, **characterized in that** it is intended for the treatment or prophylaxis of a nosocomial disease, in particular caused by an antibacterial-resistant bacterium for example in an animal, notably in a mammal, in particular in man.

**14.** Composition for use according to claim 12 or 13, **characterized in that** the nosocomial disease is selected from the group consisting of urinary tract infections, respiratory system infections, digestive system infections, central nervous system infections, skin and soft-tissue infections, bone, joint and muscle infections, vascular system infections, septic shock, diabetic foot, and eschars.

**15.** Composition for use according to any one of claims 12 to 14, **characterized in that** it is intended for the treatment or prophylaxis in immunosuppressed or immunodeficient individuals, in particular the elderly, infants, young children, patients undergoing corticosteroid therapy, transplant patients and AIDS patients.

**16.** Use of a composition as defined according to any one of claims 1 to 11, as a preservative.

**17.** Method for preparing a device, such as a catheter, including at least one step consisting in covering said surface with a composition as defined according to any one of claims 1 to 11.